(19) Europäisches Patentamt / European Patent Office / Office européen des brevets

(11) **EP 3 776 458 B1**

(12) **EUROPEAN PATENT SPECIFICATION**

(45) Date of publication and mention of the grant of the patent:
**04.05.2022 Bulletin 2022/18**

(21) Application number: **19712083.5**

(22) Date of filing: **04.03.2019**

(51) International Patent Classification (IPC):
$G06T\ 7/12^{(2017.01)}$    $G06T\ 7/00^{(2017.01)}$
$H04N\ 7/18^{(2006.01)}$    $H04N\ 5/272^{(2006.01)}$
$G06T\ 19/00^{(2011.01)}$    $G06K\ 9/00^{(2022.01)}$
$A61B\ 5/00^{(2006.01)}$    $G02B\ 21/36^{(2006.01)}$
$G06T\ 7/62^{(2017.01)}$

(52) Cooperative Patent Classification (CPC):
**G02B 21/367; G06T 7/00; G06T 7/0012;**
**G06T 7/12; G06T 7/62; G06V 20/695;**
**G06V 20/698; H04N 7/188;** A61B 5/743;
A61B 2576/00; G06T 2207/10021;
G06T 2207/10056; G06T 2207/20076;
G06T 2207/20081; G06T 2207/20084;    (Cont.)

(86) International application number:
**PCT/US2019/020570**

(87) International publication number:
**WO 2019/199392 (17.10.2019 Gazette 2019/42)**

(54) **AUGMENTED REALITY MICROSCOPE FOR PATHOLOGY WITH OVERLAY OF QUANTITATIVE BIOMARKER DATA**

MIKROSKOP MIT ERWEITERTER REALITÄT FÜR PATHOLOGIE MIT ÜBERLAGERUNG VON QUANTITATIVEN BIOMARKERDATEN

MICROSCOPE À RÉALITÉ AUGMENTÉE POUR PATHOLOGIE AVEC SUPERPOSITION DE DONNÉES QUANTITATIVES DE BIOMARQUEURS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.04.2018 US 201862656557 P**

(43) Date of publication of application:
**17.02.2021 Bulletin 2021/07**

(73) Proprietor: **Google LLC**
**Mountain View, CA 94043 (US)**

(72) Inventor: **STUMPE, Martin**
**Mountain View, CA 94043 (US)**

(74) Representative: **Robinson, David Edward Ashdown**
**Marks & Clerk LLP**
**1 New York Street**
**Manchester M1 4HD (GB)**

(56) References cited:
**US-A1- 2016 183 779**    **US-A1- 2017 169 276**

EP 3 776 458 B1

(52) Cooperative Patent Classification (CPC): (Cont.)
G06T 2207/30024; G06T 2207/30096;
G06T 2207/30242; G16H 30/40; H04N 5/272

**Description**

This application claims priority benefits of US Provisional application serial no. 62/656,557 filed April 12, 2018.

Field

This disclosure relates to the field of pathology and more particularly to an improved microscope system and method for assisting a pathologist in classifying biological samples such as blood or tissue, e.g., as containing cancer cells or containing a pathological agent such as plasmodium protozoa or tuberculosis bacteria.

Background

in order to characterize or classify a biological sample such as tissue, the sample is placed on a microscope slide and a pathologist views it under magnification with a microscope. The sample may be stained with agents such as hematoxylin and eosin (H&E) to make features of potential interest in the sample more readily seen. Alternatively, the sample may be stained and scanned with a high resolution digital scanner, and the pathologist views magnified images of the sample on a screen of a workstation or computer.

For example, the assessment of lymph nodes for metastasis is central to the staging of many types of solid tumors, including breast cancer. The process requires highly skilled pathologists and is fairly time-consuming and error-prone, especially for nodes that are negative for cancer or have a small foci of cancer. The current standard of care involves examination of digital slides of node biopsies that have been stained with hematoxylin and eosin. However, there are several limitations inherent with manual reads including reader fatigue, and intra and inter-grader reliability that negatively impact the sensitivity of the process. Accurate review and assessment of lymph node biopsy slides is important because the presence of tumor cells in the lymph node tissue may warrant new or more aggressive treatment for the cancer and improve the patient's chances of survival.

The prior art includes descriptions of the adaptation of deep learning techniques and trained neural networks to the context of digital tissue images in order to improve cancer diagnosis, characterization and/or staging. Pertinent background art includes the following articles: G. Litjens, et al., Deep learning as a tool for increasing accuracy and efficiency of histopathological diagnosis, www.nature.com/scientificreports 6:26286 (May 2016); D. Wang et al., Deep Learning for Identifying Metastatic Breast Cancer, arXiv:1606.05718v1 (June 2016); A. Madabhushi et al., Image analysis and machine learning in digital pathology: Challenges and opportunities, Medical Image Analysis 33, p. 170-175 (2016); A. Schuamberg, et al., H&E-stained Whole Slide Deep Learning Predicts SPOP Mutation State in Prostate Cancer, bioRxiv preprint http:/.bioRxiv.or/content/early/2016/07/17/064279. Additional prior art of interest includes Quinn et al., Deep Convolutional Neural Networks for Microscopy-based Point of Care Diagnostics, Proceedings of International Conference on Machine Learning for Health Care 2016.

US2017169276 A1 likewise discloses the use of learning algorithms for tissue classification purposes.

The art has described several examples of augmenting the field of view of a microscope to aid in surgery. See U.S. patent application publication 2016/0183779 and published PCT application WO 2016/130424A1. See also Watson et al., Augmented microscopy: real-time overlay of bright-field and near-infrared fluorescence images, Journal of Biomedical Optics, vol. 20 (10) October 2015.

Summary

A method is disclosed for assisting a user in review of a slide containing a biological sample with a microscope having an eyepiece. The method includes steps of (a) capturing, with a camera, a digital image of a view of the sample as seen through the eyepiece of the microscope, (b) using a first machine learning pattern recognizer to identify one or more areas of interest in the sample from the image captured by the camera, and a second machine learning pattern recognizer trained to identify individual cells, and (c) superimposing an enhancement to the view of the sample as seen through the eyepiece of the microscope as an overlay, wherein the enhancement is based upon the identified areas of interest in the sample and further comprises quantitative data associated with the areas of interest. The method includes step (d), wherein when the sample is moved relative to the microscope optics or when a magnification or focus of the microscope changes, a new digital image of a new view of the sample is captured by the camera and supplied to the machine learning pattern recognizer, and a new enhancement is superimposed onto the new view of the sample as seen through the eyepiece in substantial real time, whereby the enhancement assists the user in classifying or characterizing the biological sample.

In one embodiment the one or more areas of interest comprise cells positive for expression of a protein and wherein the quantitative data takes the form of a percent of the cells in the view as being positive for such protein expression. Examples of the protein are Ki-67, P53, and Progesterone Receptor (PR). As another example, the one or more areas of interest can take the form of individual microorganism cells and the quantitative data comprises a count of the number of microorganism cells in the view. As another example, the one or more areas of interest take the form of individual cells undergoing mitosis and wherein the quantitative data is a count of the number of cells in the view undergoing mitosis. As another example, the areas of interest are tumor cells and the quantitative data is an area measurement of the tumor cells, either absolute or relative area within a defined region in the sample.

In one possible embodiment, the quantitative data comprises a measurement, e.g., a distance measurement. As another example the measurement is an area measurement. In one specific example, the areas of interest are prostate tissue with specific Gleason grades and the quantitative measurement is relative or absolute area measurements of tumor regions having specific Gleason grades, e.g., Grade 3, Grade 4 etc.

As another example, the quantitative data can take the form of a count of the number of areas of interest in the view. For example, the machine learning model identifies individual microorganism cells in the view and displays a count of the number of such cells.

In another aspect of this disclosure, a system is disclosed for assisting a user in review of a slide containing a biological sample. The system includes a microscope having a stage for holding a slide containing a biological sample, at least one objective lens, and an eyepiece, a digital camera configured to capture digital images of a view of the sample as seen through the eyepiece of the microscope, and a compute unit comprising a machine learning pattern recognizer configured to receive the digital images from the digital camera, wherein the pattern recognizer is trained to identify regions of interest in biological samples of the type currently placed on the stage, and wherein the pattern recognizer recognizes regions of interest on a digital image captured by the camera and wherein the compute unit generates data representing an enhancement to the view of the sample as seen through the eyepiece of the microscope, wherein the enhancement is based upon the regions of interest in the sample. The system further includes one or more optical components coupled to the eyepiece for superimposing the enhancement on the field of view.

In one configuration the compute unit implements a first machine learning pattern recognizer trained to identify individual cells within the view and a second machine learning pattern recognizer trained to identify individual cells within the view which are positive for expression of a protein. The enhancement takes the form of a display of quantitative data relating to the cells which are positive for the expression of the protein. The protein can take the form of comprises Ki-67, P53, or Progesterone Receptor (PR).

In another configuration, the system includes a workstation associated with the microscope having a display providing tools for a user of the workstation to draw an annotation on an image of the view, and wherein the annotation is saved along with the image of the view in a computer memory. The workstation may further include a graphical display providing access to tools to customize the presentation of the enhancement on the field of view.

In another configuration, the compute unit implements a machine learning pattern recognizer trained to identify individual cells which are undergoing mitosis. The enhancement includes a display of quantitative data relating to the cells which are undergoing mitosis.

In another configuration, the compute unit implements one or more machine learning pattern recognizers trained to identify individual tumor cells or areas of tumor cells which are classified in accordance with specific Gleason grade (e..g, Grade 3, Grade 4 etc.). The enhancement takes the form of a display of quantitative area data relating to the tumor cells or areas of tumor cells which are classified in accordance with the specific Gleason grades.

As used in this document, the term "biological sample" is intended to be defined broadly to encompass blood or blood components, tissue or fragments thereof from plants or animals, sputum, stool, urine or other bodily substances, as well as water, soil or food samples potentially containing pathogens.

Brief Description of the Drawings

Figure 1 is a schematic diagram of an augmented reality microscope system for pathology, which is shown in conjunction with an optional connected pathologist workstation.

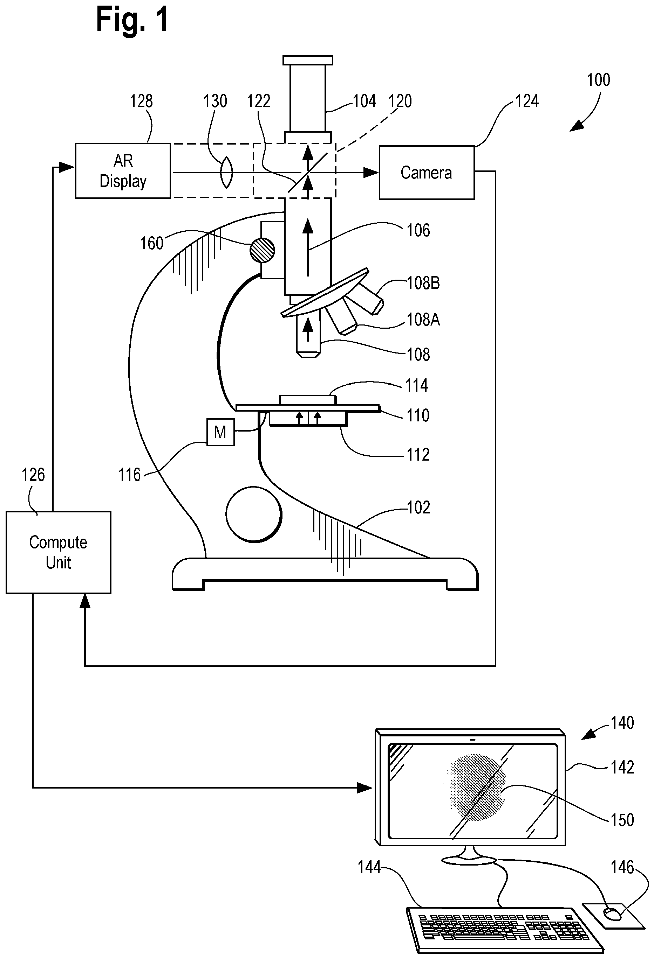

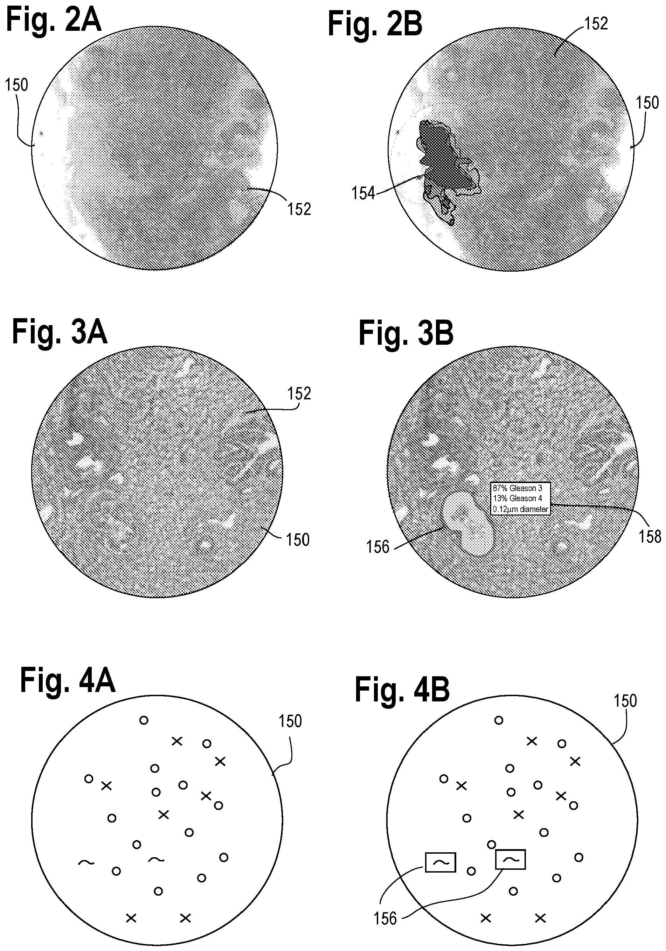

Figure 2A is an illustration of the field of view of a microscope showing a breast cancer specimen at a given magnification level, for example 10x. Figure 2B is an illustration of an augmented view seen by the pathologist using the microscope of Figure 1, with an enhancement in the form of a "heat map" superimposed on the field of view in registry will cells in the sample which are likely to be cancerous. The superimposing of the heat map in Figure 2B assists the pathologist in characterizing the sample because it directs their attention to areas of interest that are particularly likely to be cancerous. If the pathologist were to change microscope objective lenses in order to zoom in on the heat map area of Figure 2B (e.g., change to a 40X lens) a new field of view of the sample would be seen through the microscope eyepiece, a new image captured, and in substantial real time (e.g., with a second or two) a new heat map would be overlaid on the field of view (not shown) to further aid the pathologist's investigation of the sample.

Figure 3A is an illustration of the field of view of a microscope showing a prostate cancer specimen at a given magnification level, for example 10x. Figure 3B is an illustration of an augmented view seen by the pathologist using the microscope of Figure 1, with an enhancement in the form of an outline superimposed on the field of view circumscribing cells in the sample which are likely to be cancerous. The enhancement further includes a text box providing annotations, in this example Gleason score grading and tumor size data. The superimposing of the outline and annotations Figure 3B assists the pathologist in characterizing the sample because it directs their attention to areas of interest that are particularly likely to be cancerous and provides proposed scores for the sample. If the pathologist were to change focal plane position or depth (i.e., adjust focus of the microscope) in order to probe the area of interest within the outline at different depths, a new field of view of the sample would be seen through the microscope eyepiece and captured by the camera, and in substantial real time (e.g., within a second or two) a new enhancement (not shown), e.g., outline and annotation text. box, would be overlaid on the field of view to further aid the pathologist's investigation of the sample.

Figure 4A is an illustration of the field of view through the microscope of a blood sample at low magnification. Figure 4B shows the field of view of Figure 4A but with an enhancement in the form of rectangles identifying malaria parasites (plasmodium) present in the sample overlaid on the field of view to assist the pathologist in characterizing the sample.

Figure 5 is a more detailed block diagram of the compute unit of Figure 1.

Figure 6 is a flow chart showing the work flow of the system of Figure 1.

Figure 7 is a chart showing a color code or scale for interpreting an enhancement in the form of a heat map.

Figure 8 is an illustration of a machine learning pattern recognizer in the form of an ensemble of independent deep convolutional neural networks which are pre-trained on a set of microscope slide images. Each member of the ensemble is trained at a particular magnification level.

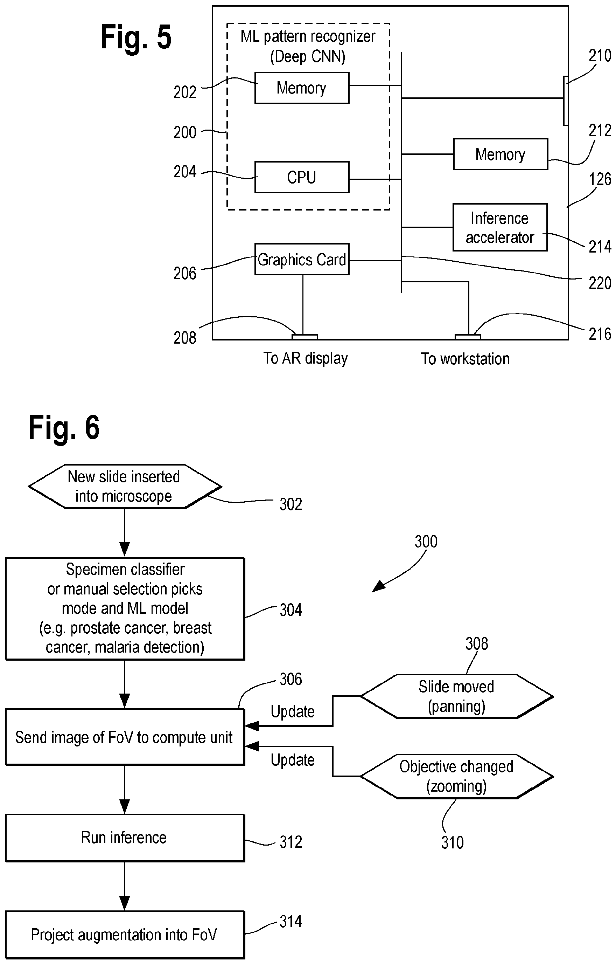

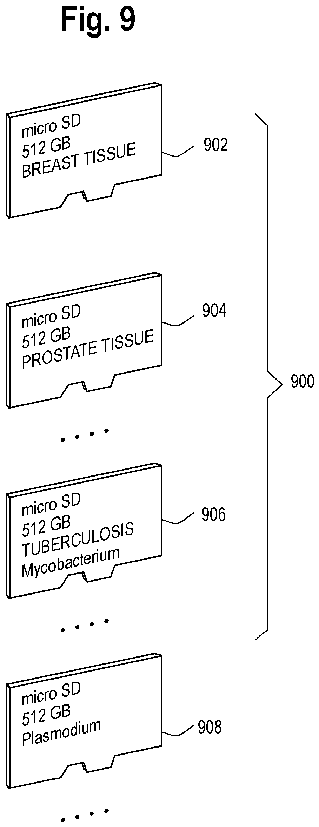

Figure 9 is an illustration of a set of portable computer storage media, each of which is loaded with code, parameters, and associated data representing an ensemble of independent deep convolutional neural networks trained on a set of microscope slide images for a particular application, such as detection of breast cancer in breast tissue, detection and characterization of cancer cells in prostate tissue, etc. A user of the system of Figure 1 who wants to augment the capability of the microscope system can obtain one or more of the media of Figure 9 and load the associated ensemble of deep convolutional neutral networks into the local compute unit of Figures 1 and 5. Alternatively, additional ensembles of deep convolutional neural networks could be downloaded from a remote data store over a network interface in the compute unit.

Figure 10 is an optics diagram of a module for projecting an enhancement or overlay into the field of view of an eyepiece of the microscope of Figure 1.

Figure 11A is an illustration of a deep learning algorithm development and model training method.

Figure 11B is an illustration of an application of the deep learning algorithm performing inference.

Figure 11C is an illustration of a software pipeline showing the sequence of operations performed on a series of images over time.

Figure 12A is a series of lymph node field of view images showing the superposition of an augmented reality enhancement in the field of view in the form of an area or border highlighted to show tumor cells in a specimen,

Figure 12B is a series of prostate field of view images showing the superposition of an augmented reality enhancement in the field of view in the form of an area or border highlighted to show tumor cells in a specimen.

Figure 13 shows several sample fields of view of lymph node specimens for metastasis detection, the first column being the augmented reality image with the overlay in the form of an outline, the second column being the heat map generated by the neural network, the third column showing the whole slide H&E image and the fourth column showing the whole slide IHC image.

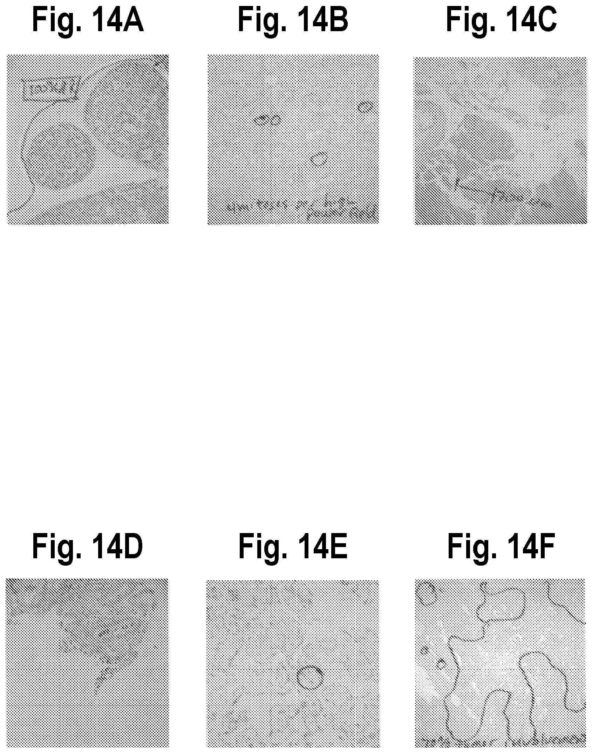

Figure 14A-F are illustrations of examples of fields of view of microscope with an overlay or enhancement in the form of quantitative measurements. Figure 14A shows a tissue sample with a border and a measurement of "100% PR" meaning all the cells within the border are positive for expression of the progesterone receptor protein. Figure 14B shows an enhancement in the form of circles drawn around cells undergoing mitosis and a quantitative report of the number of cells per high power field: "4 mitoses per high power field." Figure 14C shows a measurement or ruler showing the dimension (200 $\mu$m) of a cluster of cells. Figure 14D shows a biological sample with dark points indicating areas where the machine learning model identified the presence of individual microorganisms, in this case heliobater pylori. Figure 14E shows a biological sample with a circle indicating areas where the machine learning model identified the presence of an individual microorganism, in this case a mycobacterium. Figure 14F shows an overlay in the form of regions predicted positive for prostate cancer and a percentage of the specimen having tumor involvement (70% tumor involvement).

Figure 15 is an illustration of a workstation (which could take the form of a general purpose computing device, or tablet computer) showing an interface for drawing annotations manually on an image in the field of view, and a separate pane for providing access to tools to customize and control the rendering of annotations in the field of view.

Figure 16 is an example of an annotation superimposed on the field of view in the form of an outline surrounding areas determined by the machine learning model to be cancer/tumor cells, and a text block providing a quantitative result: "23 % tumor involvement."

Figure 17 is an example of an overlay in the form of a circle indicating the detection of a mycobacterium, e.g., tuberculosis) by a machine learning model in the field of view.

Figure 18 is an image of a field of view obtained by the camera in the microscope along with the display of an overlay in the form of biomarker quantitation, in this example the number of cells positive for expression of the protein Ki67 (98% in this example).

Figure 19 is another example of is an image of a field of view obtained by the camera in the microscope along with the display of an overlay in the form of biomarker quantitation, in this example the number of cells positive for expression of the protein P53 (49% in this example).

DETAILED DESCRIPTION

Figure 1 is a schematic diagram of an augmented reality microscope system 100 for pathology, which is shown in conjunction with an optional connected pathologist workstation 140. The system 100 includes a conventional pathologist microscope 102 which includes an eyepiece 104 (optionally a second eyepiece in the case of a stereoscopic microscope). A stage 110 supports a slide 114 containing a biological sample. An illumination source 112 projects light through the sample. A microscope objective lens 108 directs an image of the sample as indicated by the arrow 106 to an optics module 120. Additional lenses 108A and 108B are provided in the microscope for providing different levels of magnification. A focus adjustment knob 160 allows the user to change the depth of focus of the lens 108.

The microscope includes an optics module 120 which incorporates a component, such as a semitransparent mirror 122 or beam combiner/splitter for overlaying an enhancement onto the field of view through the eyepiece. The optics module 120 allows the pathologist to see the field of view of the microscope as he would in a conventional microscope, and, on demand or automatically, see an enhancement (heat map, boundary or outline, annotations, etc.) as an overlay on the field of view which is projected into the field of view by an augmented reality (AR) display generation unit 128 and lens 130. The image generated by the display unit 128 is combined with the microscope field of view by the semitransparent mirror 122. As an alternative to the semitransparent mirror, a liquid crystal display (LCD) could be placed in the optical path that uses a transmissive negative image to project the enhancement into the optical path.

The optics module 120 can take a variety of different forms, and various nomenclature is used in the art to describe such a module. For example, it is referred to as a "projection unit", "image injection module" or "optical see-through display technology." Literature describing such units include US patent application publication 2016/0183779 (see description of Figures 1, 11, 12, 13) and published PCT application WO 2016/130424A1 (see description of Figures 2, 3, 4A-4C); Watson et al., Augmented microscopy: real-time overlay of bright-field and near-infrared fluorescence images, Journal of Biomedical optics, vol. 20 (10) October 2015; Edwards et al., Augmentation of Reality Using an Operating Microscope, J. Image Guided Surgery. Vol. 1 no. 3 (1995); Edwards et al., Stereo augmented reality in the surgical microscope, Medicine Meets Virtual Reality (19997) J.D. Westward et al (eds.) IOS Press, p. 102.

The semi-transparent mirror 122 directs the field of view of the microscope to both the eyepiece 104 and also to a digital camera 124. A lens for the camera is not shown but is conventional. The camera may take the form of a high resolution (e.g., 16 megapixel) video camera operating at say 10 or 30 frames per second. The digital camera captures magnified images of the sample as seen through the eyepiece of the microscope. Digital images captured by the camera are supplied to a compute unit 126. The compute unit 126 will be described in more detail in Figure 5. Alternatively, the camera may take the form of an ultra-high resolution digital camera such as APS-H-size (approx. 29.2 x 20.2 mm) 250 megapixel CMOS sensor developed by Cannon and announced in September 2015.

Briefly, the compute unit 126 includes a machine learning pattern recognizer which receives the images from the camera. The machine learning pattern recognizer may take the form of a deep convolutional neural network which is trained on a set of microscope slide images of the same type as the biological specimen under examination. Additionally, the pattern recognizer will preferably take the form of an ensemble of pattern recognizers, each trained on a set of slides at a different level of magnification, e.g., 5X, 10X, 20X, 40X. The pattern recognizer is trained to identify regions of interest in an image (e.g., cancerous cells or tissue, pathogens such as viruses or bacteria, eggs from parasites, etc.) in biological samples of the type currently placed on the stage. The pattern recognizer recognizes regions of interest on the image captured by the camera 124. The compute unit 126 generates data representing an enhancement to the view of the sample as seen by the user, which is generated and projected by the AR display unit 128 and combined with the eyepiece field of view by the semitransparent mirror 122.

The essentially continuous capture of images by the camera 124, rapid performance of interference on the images by the pattern recognizer, and generation and projection of enhancements as overlays onto the field of view, enables the system 100 of Figure 1 to continue to provide enhancements to the field of view and assist the pathologist in characterizing or classifying the specimen in substantial real time as the operator navigates around the slide (e.g., by use of a motor 116 driving the stage), by changing magnification by switching to a different objective lens 108A or 108B, or by changing depth of focus by operating the focus knob 160. This is a substantial advance in the art and improvement over conventional pathology using a microscope.

By "substantial real time," we mean that an enhancement or overlay is projected onto the field of view within 10 seconds of changing magnification, changing depth of focus, or navigating and then stopping at a new location on the slide. In practice, as explained below, with the optional use of inference accelerators, we expect that in most cases the new overlay can be generated and projected onto the field of view within a matter of a second or two or even a fraction of a second of a change in focus, change in magnification, or change in slide position.

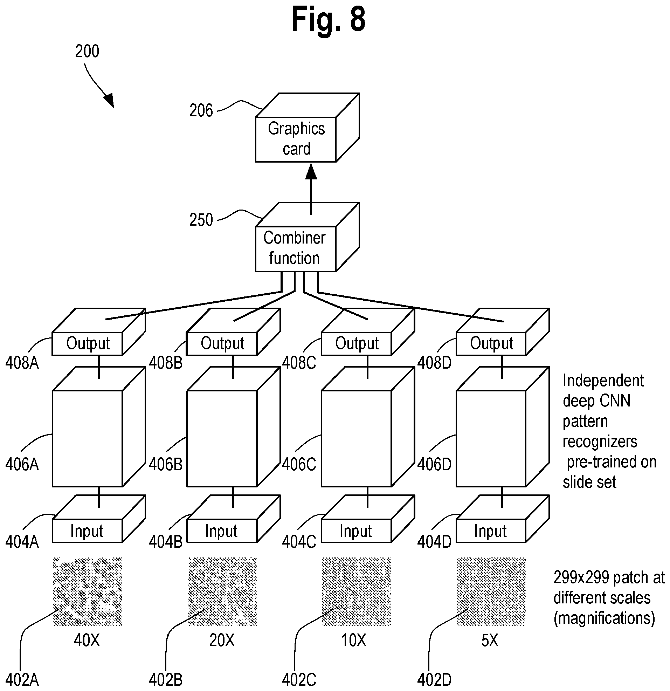

In summary then, a method is disclosed of assisting a user (e.g., pathologist) in review of a slide 114 containing a biological sample with a microscope 102 having an eyepiece 104. The method includes a step of capturing with a camera 124 a digital image of the sample as seen by the user through the eyepiece of the microscope, using a machine learning pattern recognizer (200, Figure 5, Figure 8) to identify areas of interest in the sample from the image captured by the camera 124, and superimposing an enhancement to the view of the sample as seen by the user through the eyepiece of the microscope as an overlay. As the user moves the sample relative to the microscope optics or changes magnification or focus of the microscope, a new image is captured by the camera and supplied to the machine learning pattern recognizer, and a new enhancement is overlaid onto the new view of the sample as seen through the eyepiece in substantial real time. The overlaid enhancement assists the user in classifying the biological sample.

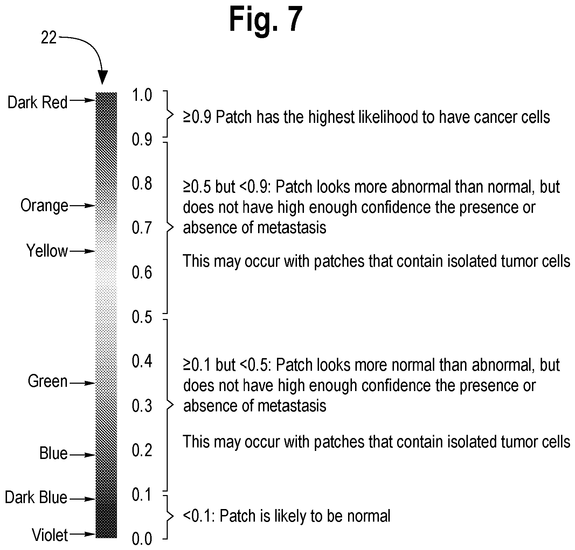

Figure 2A is an illustration of the field of view 150 of a microscope showing a breast cancer specimen 152 at a given magnification level, for example 10X. Figure 2A shows the field of view with no enhancement, as would be the case with a prior art microscope. Figure 2B is an illustration of an augmented view seen by the pathologist using the microscope of Figure 1, with an enhancement 154 in the form of a "heat map" superimposed on the field of view in registry will cells in the sample which are likely to be cancerous. The "heat map" is a set of pixels representing tissue likely to be cancerous which are colored in accordance with the code of Figure 7 to highlight areas (e.g. in red) which have a high probability of containing cancerous cells. The superimposing of the heat map 154 in Figure 2B assists the pathologist in characterizing the sample because it directs their attention to areas of interest that are particularly likely to be cancerous. If the pathologist were to change microscope objective lenses (e.g., select lens 108A in Figure 1) in order to zoom in on the heat map area 154 of Figure 2B (e.g., change to a 40X lens), a new field of view of the sample would be seen through the microscope eyepiece and directed to the camera. The camera 124 captures a new image, and in substantial real time (e.g., with a second or two) a new heat map 154 (not shown) would be generated and overlaid on the field of view to further aid the pathologist's investigation of the sample at the higher magnification.

In one possible configuration, the microscope 102 includes a capability to identify which microscope objective lens is currently in position to image the sample, e.g., with a switch or by user instruction to microscope electronics controlling the operation of the turret containing the lenses, and such identification is passed to the compute unit 126 using simple electronics so that the correct machine learning pattern recognition module in an ensemble of pattern recognizers (see Figure 8 below) is tasked to perform inference on the new field of view image. The microscope may include the automated objective identification features of PCT application serial no. PCT/US2019/012674 filed January 8, 2019.

Figure 3A is an illustration of the field of view 150 of a microscope showing a prostate cancer specimen at a given magnification level, for example 10X, as it would be in a conventional microscope without the capability of this disclosure. Figure 3B is an illustration of an augmented field of view 150 seen by the pathologist using the microscope of Figure 1, with an enhancement in the form of an outline 156 superimposed on the field of view circumscribing cells in the sample which are likely to be cancerous. The enhancement further includes a text box 158 providing annotations, in this example Gleason score grading and size measurements. In this particular example, the annotations are that 87 percent of the cells within the outline are Gleason grade 3 score, 13 percent of the cells are Gleason grade 4 score, and the tumor composed of cells of Gleason grade 4 score has a diameter of 0.12 $\mu$m.

Another possible enhancement is a confidence score that the cells of the sample are cancerous. For example, the enhancement could take the form of a probability or confidence score, such as 85% confidence that the cells in the outline are Gleason Grade 3, and 15% confidence that the cells in the outline are Gleason Grade 4. Additionally, the measurement (0.12 $\mu$m) could be the diameter of the whole outlined region.

The superimposing of the outline and annotations Figure 3B assists the pathologist in characterizing the sample because it directs their attention to areas of interest that are particularly likely to be cancerous and provides proposed scores for the sample. If the pathologist were to change depth of focus of the microscope in order to probe the area of interest within the outline 156, a new field of view of the sample would be seen through the microscope eyepiece and captured by the camera 124, and in substantial real time (e.g., within a second or two) a new enhancement, e.g., outline and annotation text box, would be overlaid on the field of view (not shown) to further aid the pathologist's investigation of the sample. The system of Figure 1 optionally includes the ability for the pathologist to turn on or off the enhancement projections, e.g., by providing controls for the system on the attached workstation 140 of Figure 1, providing a simple user interface on the compute unit 126, or by a foot switch that turns on and off the AR display unit 128.

Figure 4A is a hypothetical illustration of the field of view 150 through the microscope of a blood sample at low magnification, as it would be seen in a conventional microscope. The view includes various blood fragments (red and white blood cells) and components such as platelets. Figure 4B shows the same field of view of Figure 4A but with an enhancement in the form of rectangles 156 identifying malaria parasites (plasmodium) present in the sample overlaid on the field of view to assist the pathologist in characterizing the sample, in this case as positive for malaria.

Table 1 below lists optical characteristics of a typical microscope for pathology and the digital resolution of a camera 124 which could be used in Figure 1.

Table 1

| Objective | Field of View (diameter) | Digital resolution ($\mu$m per pixel)* | Used for |
|---|---|---|---|
| 4x | 4.5 mm | 3.5 | Low power (screening) |
| 10x | 1.8 mm | 1.4 | Low power (tissue morphology) |
| 20x | 0.9 mm | 0.7 | Medium power |
| 40x | 0.45 mm | 0.35 | High power (cellular detail) |
| 100x | 0.18 mm | 0.14 | Special purpose (cytology, e.g. malaria) needs special optics with oil immersion |
| * based on an 16MP camera | | | |

Figure 5 is a block diagram of one possible form of the compute unit 126 of Figure 1. Essentially, in one possible configuration the compute unit is a special purpose computer system designed to perform the required tasks of the system of Figure 1, including performing inference on captured images, generation of digital data for overlays for the field of view, optional inference acceleration to perform the inference operations sufficiently quickly to enable substantial real time display of enhancements, as well as the capability to load additional machine learning models (pattern recognizers) to support additional pathology tasks.

In Figure 5, the compute unit includes a deep convolutional neural network pattern recognizer 200 in the form of a memory 202 storing processing instructions and parameters for the neural network and a central processing unit 204 for performance of inference on a captured image. The module may also include a graphics card 206 for generating overlay digital data (e.g. heat maps, annotations, outlines, etc.) based on the inference results from the pattern recognizer 200. A memory 212 includes processing instructions for selecting the appropriate machine learning model based on the current magnification level, and coordinate sharing of the image of the field of view with a remote workstation 140 (Figure 1), and other tasks as explained herein. The compute unit may also include an inference accelerator 214 to speed up the performance of inference on captured images. The compute unit further includes various interfaces to other components of the system including an interface, not shown, to receive the digital images from the camera, such as a USB port, an interface (e.g., network cable port or HDMI port) 208 to send digital display data to the AR display unit 128, an interface (e.g., network cable port) 216 to the workstation 140, and an interface 210 (e.g., SC card reader) enabling the compute unit to receive and download portable media containing additional pattern recognizers (see Figure 9) to expand the capability of the system to perform pattern recognition and overlay generation for different pathology applications. A high speed bus 220 or network connects the modules in the compute unit 126. In practice, additional hard disk drives, processors, or other components may be present in the compute unit, the details of which are not particularly important.

In another possible configuration, the compute unit 126 could take the form of a general purpose computer (e.g., PC) augmented with the pattern recognizer(s) and accelerator, and graphics processing modules as shown in Figure 5. The personal computer has an interface to the camera (e.g., a USB port receiving the digital image data from the camera), an interface to the AR projection unit, such as an HDMI port, and a network interface to enable downloading of additional pattern recognizers and/or communicate with a remote workstation as shown in Figure 1.

In use, assuming multiple different pattern recognizers are loaded into the compute unit, an automatic specimen type detector or manual selector switches between the specimen dependent pattern recognition models (e.g. prostate cancer vs breast cancer vs malaria detection), and based on that the proper machine learning pattern recognizer or model is chosen. Movement of the slide to a new location (e.g., by use of a motor 116 driving the stage) or switching to another microscope objective 108 (i.e. magnification) triggers an update of the enhancement, as explained previously. Optionally, if only the magnification is changed, an ensemble of different models operating at different magnification levels (see Figure 8) performs inference on the specimen and inference results could be combined on the same position of the slide. Further details on how this operation could be performed are described in the pending PCT application entitled "Method and System for Assisting Pathologist Identification of Tumor Cells in Magnified Tissue Images", serial no. PCT/US17/019051, filed February 23, 2017.

Another option is that the compute unit could know the current magnification from the microscope by means of simple electronic communication from the microscope to the compute unit. The microscope monitors which lens is placed by the user into the optical path and communicates the selection to the compute unit.

Deep convolutional neural network pattern recognizers, of the type used in the compute unit of Figure 5 shown at 200, are widely known in the art of pattern recognition and machine vision, and therefore a detailed description thereof is omitted for the sake of brevity. The Google Inception-v3 deep convolutional neural network architecture, upon which the present pattern recognizers are based, is described in the scientific literature. See the following references: C. Szegedy et al., Going Deeper with Convolutions, arXiv: 1409.4842 [cs.CV] (September 2014); C. Szegedy et al., Rethinking the Inception Architecture for Computer Vision, arXiv:1512.00567 [cs.CV] (December 2015); see also US patent application of C. Szegedy et al., "Processing Images Using Deep Neural Networks", serial no. 14/839,452 filed August 28, 2015. A fourth generation, known as Inception-v4 is considered an alternative architecture for the pattern recognizers 306. See C. Szegedy et al., Inception-v4, Inception-ResNet and the Impact of Residual Connections on Learning, arXiv:1602.0761 [cs.CV] (February 2016). See also U.S. patent application of C. Vanhoucke, "Image Classification Neural Networks", serial no. 15/395,530 filed December 30, 2016.

Additional literature describing deep neural network pattern recognizers include the following G. Litjens, et al., Deep learning as a tool for increasing accuracy and efficiency of histopathological diagnosis, www.nature.com/scientificreports 6:26286 (May 2016); D. Wang et al., Deep Learning for Identifying Metastatic Breast Cancer, arXiv:1606.05718v1 (June 2016); A. Madabhushi et al., Image analysis and machine learning in digital pathology: Challenges and opportunities, Medical Image Analysis 33 p 170-175 (2016); A. Schuamberg, et al., H&E-stained Whole Slide Deep Learning Predicts SPOP Mutation State in Prostate Cancer, bioRxiv preprint http://.bioRxiv.or/content/early/2016/07/17/064279,

Sources for training slides for training the deep neural network pattern recognizer 200 can be generated from scratch by whole slide scanning of a set of slides of the type of samples of interest. For example, slide images for training can be obtained from Naval Medical Center in San Diego, California (NMCSD) and publicly available sources such as from the CAMELYON16 challenge and The Cancer Genome Atlas (TCGA). Alternatively, they could be generated from a set of images of different slides captured by the camera of Figure 1.

Digital whole slide scanners and systems for staining slides are known in the art. Such devices and related systems are available from Aperio Technologies, Hamamatsu Photonics, Philips, Ventana Medical Systems, Inc., and others. The digital whole slide image can be obtained at a first magnification level (e.g. 40X), which is customary. The image can be upsampled or downsampled to obtain training images at other magnifications. Alternatively, the training slides can be scanned multiple times at different magnifications, for example at each magnification level offered by conventional manually-operated microscopes.

Inference speed

In some implementations it may be possible to perform inference on a digital image that is the entire field of view of the microscope. In other situations, it may be desirable to perform inference on only a portion of the image, such as several 299x299 rectangular patches of pixels located about the center of the field of view, or on some larger portion of the field of view.

Using an Inception v3-based model with 299x299 pixel input size and a 16MP camera, a dense coverage of a spherical area of the optical FoV (2700 pixels diameter) requires ~120 patch inferences. If inference is run only for the center third (increasing inference granularity, and using the other two third as context), it will require ~1200 inference calls. Additional inference calls might be required if one adds rotations and flips, or ensembling.

Table 2 lists the number of inference calls and inference times using conventional state of the art graphics processing units and inference accelerators.

Table 2

| Configuration | # inference calls for FoV | inference time (GPU)* | inference time (accelerator)** |
|---|---|---|---|
| Dense coverage, Inception V3 (baseline) | 120 | 0.8 sec | 2 msec |
| Dense coverage, inference on center third (stride ⅓) | 1200 | 8 sec | 0.02 sec |
| 8 Rotations and flips | 9600 | 64 sec | 0.17 sec |

(continued)

| Configuration | # inference calls for FoV | inference time (GPU)* | inference time (accelerator)** |
|---|---|---|---|
| Ensembling (5 models) | 48000 | 320 sec | 0.85 sec |

\* assuming 150 inferences per second, Inception-v3
\*\* assuming 56000 inferences per second with an inference accelerator system

Assuming a camera 124 operates at 30 frames per second (fps) for a seamless substantial near real time experience, a dense coverage with a reasonable combination of rotation, flips, and ensembling is possible.

Inference accelerator (214, Figure 5)

Inference accelerators, also known as artificial intelligence (AI) accelerators, are an emerging class of microprocessors or coprocessors which are designed to speed up the process of performing inference of input data sets for pattern recognition. These systems currently take the form of a combination of custom application-specific integrated circuit chips (ASICs), field programmable gate arrays (FPGAs), graphics processing units (GPUs) and general purpose computing units. In some applications of the system of Figure 1 it may be desirable to include an inference accelerator in the compute unit 126, as shown in Figure 5. Inference accelerators are described in the art, see Jonathon Ross, et al., U.S. patent application publication 2016/0342891 entitled "Neural Network Processor", and currently available on the market such as the NVidia TM and Tesla TM P40 and P4 GPU Accelerators and the Intel TM Deep Learning Inference Accelerator.

In a simple implementation, the system of Figure 1 could just use a USB camera output plugged into a standard PC (compute unit 126) which performs the pattern recognition and outputs the overlay graphic (enhancement) via a graphic card output interface (e.g., HDMI) to the AR display device. The inference can be done by a graphics processing unit (GPU) in the standard PC. In this configuration, an on-device inference accelerator would be optional and not necessary. In the event that the need arises for faster inference, the computer could be augmented later on with an off-the shelf inference accelerator as a plug-in module.

Generation of Enhancement

The generation of the enhancement to project onto the field of view can be performed as follows:

1) the machine learning pattern recognizer 200 in the compute unit 126 runs model inference on the field of view, to create tumor probability per region (using cancer detection as an example here).
2a) heatmap: the tumor probability for each image patch in the field of view is translated into a color value (e.g. RGB), and those color values are stitched together to create a heatmap. This task can be performed by the graphics card 206.
2b) polygon outline: the tumor probabilities are thresholded at a certain score (e.g. probability > 50%), and the boundary of the remaining region (or regions, if there are several not connected regions) form the polygon outline. Again this task can be performed by the graphics card 206.
3) the digital image data from step 2A or 2B is translated into an image on a display by the AR display unit 128, that is then projected into the optical path by lens 130 and semi-transparent mirror 120.

Additionally, the graphics card 206, either alone or with outputs from the machine learning pattern recognizer can generate Gleason score grading, annotations etc. for including in the digital enhancement data and provide such additional enhancements to the AR display module 128.

Communication of the microscope with a computer about the location on the slide.

In practice, in some situations it may be useful to perform a whole slide scan of the specimen slide in addition to pathologist use of the microscope system of Figure 1. In this situation, the whole slide scan may be resident on the workstation 140 (or shared by both the workstation 140 and the compute unit 126). A number of possible uses may be made of the enhancement to the field of view, including:

1. highlighting of the microscope current field of view (FoV) on the whole slide image (e.g. for teaching purposes). Localization of the FoV could be done either via image registration of the microscope image onto the whole slide image, or by use of the motor 116 driving the microscope stage 110 with the motor coordinates mapped onto the whole slide image coordinates.

2. automatic navigation of the microscope FoV to a designated area on the slide. For example, the microscope could operate in a "pre-scan" mode in which the motor 116 drives the microscope slide to a series of X-Y positions and obtains low magnification images with the camera at each position. The images are passed to the machine learning pattern recognizer in the compute unit 126 and the pattern recognizer identifies those images from respective positions that contain areas of interest (e.g., cells likely to be cancerous). Then, during use by the pathologist, the motor 116 could be operated to drive the slide to those positions and the operator prompted to investigate the field of view at each position and the field of view augmented with suitable enhancements (heat maps, outlines, etc.). In this embodiment, the compute unit may operate in conjunction with a user interface for the microscope to aid the pathologist work flow. Such user interface could be incorporated in the microscope per se or be presented in the display 142 of the workstation 140. For example, in Figure 1 the workstation 140 includes a display 142 which displays the current field of view 150 of the microscope. By using the mouse 146 or keyboard 144 the pathologist could enter commands on the workstation to cause the microscope stage motor 116 to step through a sequence of positions on the slide containing areas of interest. The identification of areas of potential interest at low magnification could be performed on the whole slide image, and the positions of areas of potential interest translated to slide coordinates using a mapping of motor coordinates to slide coordinates.

3. transfer of labels and annotations from the whole slide image to the microscope image

A whole slide image of the specimen slide obtained by a whole slide scanner can be provided with labels or annotations for various objects of interest in the image. Because it is possible to obtain registry between the whole slide image and the slide on the motorized stage 110 (e.g., from a mapping of motor 116 positions to whole slide image coordinates), it may be possible transfer the labels and annotations to the microscope image seen through the eyepiece. This is possible by providing the labels and annotations to the graphics card 206 in the compute unit, and then providing the digital data of such labels and annotations to the AR display unit 128 when the motor drives the slide to the coordinates where such labels and annotations exist.

The method of obtaining registration between the whole slide image and the slide on the microscope could be implemented as an algorithmic solution, or by using computer vision approaches, such as image registration, to locate the region of the whole slide image that corresponds to the camera image.

4. Output of the field of view along with the prediction to a local storage, for usage in e.g. a pathology report.

In practice, it may be desirable for the pathologist to make records of their work in characterizing or classifying the sample. Such records could take the form of digital images of the field of view (with or without enhancements) which can be generated and stored (e.g., in the memory 212 of the compute unit) and then transmitting them via interface 216 to the attached pathology workstation 140. The workstation software will typically include workflow software that the pathologist follows in performing a classification or characterization task on a sample and generating a report. Such software includes a tool, e.g., icon or prompts, which permit the pathologist to insert into the report the stored digital images of the field of view and relevant annotations or enhancements which are stored in the memory 212.

Further optional features may be included in the system.

A. Output port for displaying field of view on a monitor

The compute unit includes an interface or port 216 for connecting the compute unit to the attached peripheral pathologist workstation 140. This interface allows the field of view captured by the camera and any enhancement generated by the graphics card to be transmitted to the monitor 142 of the workstation 140.

B. On demand a connected monitor displays image regions that are similar to the one in the current field of view, with annotations etc.

In one possible configuration, the monitor 142 of the workstation 140 displays image regions from other slides (e.g., from other patients) that are "similar" to the one in the current field of view, along with any enhancements or annotations which may exist for the other slide(s). In particular, the workstation 140 may include a memory loaded with digital image data of a set of other slides from other patients, and potentially hundreds or thousands of such slides. The workstation may include a pattern recognizer which performs pattern recognition of the field of view of the slide on the microscope on all of such other digital slide images and selects the ones that are closest to the field of view. Fields of view (i.e., portions of the selected digital slides stored in memory) can be presented on the display 142 of the workstation 140 alongside the current field of view through the microscope 100. Each of the slides stored in memory on the workstation is associated with metadata such as the patient diagnosis, date, treatment, outcome or survival data after treatment, age, smoker status, etc. The display of the fields of view of the selected digital slides can be augmented with the display of the metadata.

Examples of Enhancements

1. heat map

Figure 2B shows an example of a "heatmap" in the form of an overlay of colored pixels which identify areas of particular interest, e.g., likely to contain cancer cells.

Heatmaps assist the pathologist in reviewing a slide by presenting to the pathologist an overlay on the field of view in which discrete areas (i.e., groups of pixels) of the slide which have a high probability of containing tumor cells are indicated in a particular color, e.g., dark red. Conversely, areas in the field of view with relatively low probability of containing tumor cells could be left alone or rendered in a contrasting color, e.g., blue or violet. The heatmap image can be accompanied by a list of different regions, where there are groups of cells with a high probability of containing tumor cells.

In one embodiment, the scores for small groups of pixels ("patches") in the digital slide image captured by the camera 124 range from 0.0 to 1.0. The areas of the heatmap 20 with the highest scores are shown as dark red, whereas the areas with the areas with the lowest scores are either left alone (not enhanced) or shown in another contrasting color, such as violet. The code 22 of Figure 7 essentially uses the visible spectrum (i.e., colors of the rainbow) to assign colors to tumor probability scores. However, it would be possible to use only a portion of the visible spectrum, for example only generate an enhancement of pixels which have tumor probability likelihood of greater than 0.5. Moreover, in yet another possible alternative embodiment only degrees of grayscale could be used for the code, e.g., with white corresponding to a score of 0, black corresponding to score of 1, and degrees of gray making up the values between 0 and 1. For example, single color (e.g., green) could be used and opacity (grayscale) can be used to encode tumor probability.

Further details on the generation and calculation of heatmaps and tumor probability scores are described in the pending PCT application "Method and System for Assisting Pathologist identification of Tumor Cells in Magnified Tissue images", serial no. PCT/US17/019051 filed February 23, 2017.

2. outlines of regions of interest and annotations

Figure 3B shows an example of the outline of a region of interest, which can be generated and projected on the field of view as described above. The outlines can be accompanied by textual matter (annotations) such as Gleason score, measurements of size, e.g., tumor diameter, cancer likelihood prediction, cell counts or other relevant pathology information. The display of size measurement data, e.g., "tumor diameter 2mm", is possible because the compute unit knows the current objective lens power and hence can translate pixels of image data into physical units. Such measurements can trigger additional labels or annotations, such as "micrometastasis" vs "macrometastasis". The annotations could also include statistics, such as the % of the image positive for cancer cells and the % of the image negative for cancer cells, and confidence or probability scores.

3. rectangles identifying objects

Figure 4B shows an example of the use of rectangles or bounding boxes placed around objects of interest. This approach may be used for example in the identification of bacteria (e.g., tuberculosis), protozoa (e.g., plasmodium), eggs from parasites, or other pathogens in food, blood, water or other types of biological samples. The rectangles could be accompanied by additional information such as annotation like size, confidence or probability scores, species identification, etc., depending on the application.

4, Quantitative data

See the section below.

Workflow

Figure 6 is a flow chart showing the workflow using the system of Figure 1. At step 302, the user inserts a new slide 114 onto the microscope stage 110. At step 302, a specimen classifier or manual selection (e.g., by use of the attached workstation 140 or by user interface controls on the microscope or on the compute unit) selects the pattern recognition mode (e.g., breast cancer, prostate cancer, malaria) according to the type of specimen on the slide and the relevant machine learning pattern recognizer in the compute unit is flagged for operation.

At step 306 an image of the field of view is captured by the digital camera 124 and send to the compute unit 126. If the operator moves the slide (e.g., by operation of the stage motor 116 in a panning mode) a new image of the field of view is captured by the camera. Similarly, if the operator changes the objective lens 108 (e.g., to zoom in or out) a new image is captured. The new images are sent to the compute unit 126. (In practice, the camera 124 could be operated at a continuous frame rate of say 10 or 30 frames per second and the updating of the field of view in the compute unit could be essentially continuous and not merely when either stage position or objective lens are changed.)

At step 312 the image of the field of view is provided as input to the relevant machine learning pattern recognizer 200 in the compute unit 126 (Figure 5) to perform inference. As a practical matter, step 312 may be performed repeatedly, in synchrony with the frame rate of the camera 124.

At step 314 the graphics card or GPU 206 in the compute unit 126 generates digital image data corresponding to the enhancement or augmentation relevant to the sample type and this digital image data is provided to the AR display unit 128 for projection onto the field of view for viewing by the pathologist in the eyepiece 104.

The compute unit may include controls (e.g., via the attached workstation) by which the user can specify the type of annotations or enhancements they wish to see projected onto the field of view, thereby giving the user control as to how they wish the microscope to operate in augmented reality mode. For example, the user could specify enhancements in the form of heat map only. As another example, if the specimen is a blood sample, the user could specify enhancements in the form of rectangles identifying plasmodium present in the sample. In a prostate sample, the user can specify boundaries our outlines surrounding cells which a Gleason score of 3 or more, as well as annotations such as shown and described previously in Figure 3B. As another example, the user may be provided with a switch (such as a foot switch) to turn on and off the projection and thus display of the enhancements in the microscope field of field of view.

Ensemble of machine learning pattern recognizers

It will be noted that the system of Figure 1 is designed to be used in conjunction with a microscope offering several different objective lenses and magnification levels. Typically, a particular pattern recognizer or machine learning model is trained on a set of training slides at a particular magnification level. Accordingly, to accommodate the possibility of the user changing objective lenses during inspection of a given sample, a preferred embodiment of the compute unit includes an ensemble of pattern recognizers, each trained on image data at different magnification levels. For example, in Figure 8, there is shown an ensemble of four different pattern recognizers (406A, 406B, 406C and 406D). Each of the pattern recognizers takes the form of a deep convolutional neural network trained on a set of digital slide images at a particular magnification. For example, pattern recognizer 406A is trained on 40X magnification slide images. Pattern recognizer 406B is trained on 20X magnification slide images. Pattern recognizer 406C is trained on 10X magnification slide images. Pattern recognizer 406D is trained on 5X magnification slide images. Ideally, each of the magnification levels the pattern recognizers are trained at correspond to the magnification levels which are available on the microscope of Figure 1. This is not essential, because if there is a mismatch between microscope magnification and training slide magnification the microscope image captured by the camera 124 could be upsampled or downsampled to correspond to the magnification level of the pattern recognizer.

In operation, a patch (i.e., a portion of the microscope FoV, such as a 299x299 rectangular patch of pixels) 402A, 402B, 402C or 402D is provided as an input 404A, 404B, 404C or 404D to the relevant pattern recognizer 406A, 406B, 406C, 406D depending on the current objective lens being used on the microscope. In a heat map application, the score for a patch of pixels between 0 and 1 is generated as the last layer of the neural network pattern recognizers 406A, 406B, 406C, 406D, in the form of a multinomial logistic regression, which generates a prediction, in the form of a probability of between 0 and 1, of which of the classes (here, healthy vs tumor) the input data (patch) belongs to. Multinomial logistical regression is known in the art of supervised learning and optimization, and is sometimes referred to as "Softmax Regression." A tutorial found on the web, http://ufldl.stanford.edu/tutorial/supervised/SoftmaxRegression/ provides further details. The output 408A, 408B, 408C, 408D is thus the score for the patch of pixels.

In one configuration, the process of generating a score for a patch of pixels is performed for all of the patches forming the field of view of the microscope. The outputs 408A, 408B, 408C 408D are provided to the graphics card (GPU) 206 in the compute unit to generate data representing the augmentation, in this example the heat map. In the situation where the stage remains stationary but the user changes magnification, then two of the members of the ensemble shown in Figure 8 can be used to generate heat maps, one for the first magnification and one for the second magnification. In theory it is possible to combine the outputs of the members of the ensemble. Thus, in one variation, where multiple members of the ensemble are used, for example where there was a change in the focus plane, multiple outputs 408A, 408B, 408C or 408D are supplied to a combiner function 250 which combines the outputs and sends the data to the graphics card. Further details are described in the previously cited PCT patent application filed February 23, 2017.

It will also be appreciated that the compute unit preferably includes an ensemble of pattern recognizers trained on a set of microscope slide images at different magnification levels for each of the pathology applications the microscope is used for (e.g., breast cancer tissue, lymph node tissue, prostate tissue, malaria, etc.), as indicated in Figure 8.

Portable media with machine learning pattern recognizers

In one embodiment, the compute unit 126 of Figures 1 and 5 includes a hardware configuration to receive and store locally new trained pattern recognizers/machine learning models, for different types of biological samples or applications of the microscope. This configuration is shown for example in Figure 5 as a SD card interface 210 which allows individual SD cards containing machine learning models to be inserted into the compute unit and the content downloaded and stored in the memory 202. Figure 9 shows an example of a set 900 of SD cards, including cards 902, 904, 906 and 908. Each card contains the model parameters, filter coefficients, executable code and other details of a machine learning pattern recognizer for a particular pathology application, such as identification of cancer cells in breast tissue (card 902), identification of cancer cells in prostate tissue (904), identification of tuberculosis mycobacterium in a blood sample (card 906), identification of Plasmodium protozoa in blood samples for malaria detection (card 908). In this design, the compute unit could be provided for example as standard equipment with a machine learning model for a common pathology application, such as for example cancer cell detection in a pap smear, and the user could obtain from a provider or source an additional card or set of cards 900 to enhance the capabilities of the microscope for other pathology applications. In this manner the laboratory operating the microscope can tailor their needs for augmented reality in a microscope to particular pathology applications as needed or as the market dictates. The laboratory need not create the models, rather a service provider could create them separately from a set of training slide images, validate the models to insure robustness and generalizability, and then create portable storage media such as SD cards containing such models and provide them to customers on an as-needed basis.

While SD cards are illustrated in Figure 9, other physical formats of memory devices capable of storing machine learning pattern recognition models could of course be used, including those available currently and those generated in the future. It is also possible for the compute unit to connect to a computer network and download additional machine learning models (or ensembles of models) over a computer interface such as the interface 216 (Figure 5).

Specific applications

While several specific applications of the microscope for pathology review have been described, including breast cancer detection, prostate cancer detection, identification of pathogens (e.g., plasmodium, tuberculosis, malaria parasites, eggs of parasites) etc., it will be appreciated that other applications in the field of pathology are of course possible. Additionally, the principles of the system of Figure 1 could be extended to other applications of microscopy, such as quality control inspection of small parts, food safety or inspection, water quality monitoring, and the like.

Stand-alone system

The microscope system of Figure 1 with local compute unit and pattern recognition model(s) is ideally suited as a local, stand-alone system. As long as it has available a suitable power supply for the electronics shown in Figure 1 and 5, it can be considered portable and used in remote locations. In its most basic form, it does not require any internet or other network connection, and the attached peripheral workstation 140 is not absolutely necessary. The compute unit could come with its own attached user interface (not shown) or controls to turn augmentation on or off, select models, change to the appropriate machine learning model for the particular magnification chosen, and any other ancillary tasks. The design of the user interface could take any suitable form, such as simple touch screen and icons to guide the user to provide appropriate selections.

Networked Configuration

In another configuration, the system of Figure 1 could be implemented in a networked environment where the compute unit is connected to remote servers, e.g. to obtain new machine learning models or to perform the inference, or inference acceleration, on a separate platform. For example, some of the processing tasks described previously in conjunction with the display of the remote workstation 140 could be performed either locally on the workstation or on a networked computer remote from both the compute unit and the workstation.

Motor-driven Stage 110/116

The incorporation of a motor driven stage 110 (which is common in pathology microscopes) allows for additional functions to be formed to further assist the pathologist. For example, the motor 116 could drive the slide to a sequence of positions to capture low magnification images with the camera of the entire slide. The low magnification images are then supplied to a machine learning pattern recognizer in the compute unit trained at low magnification levels to provide preliminary detection of suspicious regions (e.g., regions likely containing cancer cells or likely to contain tuberculosis mycobacteria. Then, the microscope stage could be driven automatically in a series of steps to those fields containing potentially relevant areas. The incremental positioning of the slide could be executed upon command of the user, e.g., via controls for the microscope or via the user interface of the attached workstation.

An exhaustive search of the whole slide at 40X for areas of interest in a short amount of time is not currently feasible with current technology. However, the use of a low magnification model able to detect suspicious regions at low magnification and then only zoom in on demand is currently feasible using the system of Figure 1.

Model training

Images obtained from the camera 124 may, in some implementations, be different in terms of optical quality or resolution than images from whole slide scanners on which the machine learning pattern recognizers are trained. The quality of the digital camera 124 and associated optical components has a lot to do with this, and ideally the quality of the digital camera and associated optics is the same as, or nearly the same as, the quality of the optical components and camera used for capturing the training slide images. While the image resolution should be comparable, the images from the microscope camera 124 are likely to have some artifacts such as geometric distortion that are absent or less frequently present in the whole slide scanner training images. Collecting microscope-specific training images for training new models is in theory possible. However it is not a particularly scalable solution. A more practical solution is make sure the whole slide image-based pattern recognition models generalize to the microscope images captured by the camera 124. If generalization with the default models is not acceptable, it should be possible to generate artificial training data from whole slide image scans that "look like" their corresponding microscope camera images. Such artificial training data can be generated by introducing parametric deformations to the whole slide image scan images and using the deformed images for training. Examples of such parametric deformations include warping, adding noise, lowering resolution, blurring, and contrast adjustment.

An alternative is to use the camera of a microscope to generate a large number of training images from a multitude of slides, and then use such images to train the models instead of images obtained from a whole slide scanner.

Another alternative training a generative adversarial network (GAN) to produce the images for training the machine learning pattern recognizers.

Further considerations

The image quality of the camera 124 of Figure 1 is an important consideration. Since a camera live stream will often be the image source, an investigation should be performed on how the image quality of the images in the live stream compares to the still images (typically high quality) obtained from a whole slide scanner and used for training, and how that affects model performance.

One particular challenge is that the optical resolution of the human eye is much higher than that of current digital cameras. For instance, in order to detect a tiny metastasis, a machine learning model might require zooming in further (switching to higher power objectives) than a human might need to for the same metastasis. One way of addressing this is prompting the user to switch to high (or higher) magnification levels when they are viewing areas of potential interest and then generating new enhancements at the higher power. Another approach is to use an ultra-high resolution camera such as the Cannon 250 megapixel CMOS sensor.

As noted above, the optical component 120 including the semi-transparent mirror 122 should be placed in the optical path so that it renders the best visual experience. In one possible configuration the microscope may take the form of a stereoscopic microscope with two eyepieces and it may be possible to project the enhancement into the field of view of one or both of the eyepieces.

Another consideration is making sure the eye sees the enhancement or overlay on the field of view with the same registration as the camera. This could be performed using fiduciary markers which are present in the field of view and the image captured by the camera.

It is also noted that labels which may be present on whole slide images of the slide under examination can be transferred to the camera images and projected into the field of view, e.g., using image registration techniques, as described previously,

Changes to the optics by the user (e.g. focusing, diopter correction) will affect the image quality on the camera image and the displayed image. The camera images need to remain sharp and high quality so that inference can be performed In one possible configuration, the compute unit includes an image quality detector module that assesses when the image is good enough to perform inference. If the image is not of sufficient quality the user could be prompted to make appropriate correction, such as adjust the focus or make other optical adjustments to the microscope.

It was also noted previously that the augmented reality microscope of this disclosure is suitable for other uses, such as inspection or quality control, e.g., in manufacturing of electronic components or other products where the inspection occurs via a microscope. Thus, as an additional aspect of this disclosure, a method for assisting a user in review of a object (e.g., manufactured object) with a microscope having an eyepiece has been disclosed, including the steps of (a) capturing, with a camera, a digital image of the object as seen by a user through the eyepiece of the microscope, (b) using a machine learning pattern recognizer to identify areas of interest (e.g., defects) in the object from the image captured by the camera, and (c) superimposing an enhancement to the view of the object as seen by the user through the eyepiece of the microscope as an overlay. As the user moves the sample relative to the microscope optics and then stops or changes magnification or focus of the microscope, a new digital image is captured by the camera and supplied to the machine learning pattern recognizer, and a new enhancement is superimposed onto the new view of the object as seen through the eyepiece in substantial real time, whereby the enhancement assists the user in classifying or characterizing the object. The features of the appended claims are deemed to be applicable to this variation wherein instead of a biological sample on a slide an object (e.g., manufactured object, computer chip, small part, etc.) is viewed by the microscope and the camera captures images of the object as seen in the microscope field of view.

An aspect may also provide a system assisting a user in review of a slide containing a biological sample, comprising, in combination: a microscope having a stage for holding a slide containing a biological sample, at least one objective lens, and an eyepiece, a digital camera capturing magnified digital images of the sample as seen through the eyepiece of the microscope, a compute unit comprising a machine learning pattern recognizer which receives the digital images from the digital camera, wherein the pattern recognizer is trained to identify regions of interest in biological samples of the type currently placed on the stage, and wherein the pattern recognizer recognizes regions of interest on the digital image captured by the camera and wherein the compute unit generates data representing an enhancement to the field of view of the sample as seen by the user through the eyepiece; and one or more optical components coupled to the eyepiece for superimposing the enhancement on the field of view; wherein the camera, compute unit and one or more optical components operate such that as the user moves the sample relative to the microscope optics and then stops or changes magnification or focus of the microscope, a new digital image is captured by the camera and supplied to the machine learning pattern recognizer, and a new enhancement is superimposed onto the new field of view of the sample as seen through the eyepiece in substantial real time.

Additional Embodiments

Opto-mechanical design

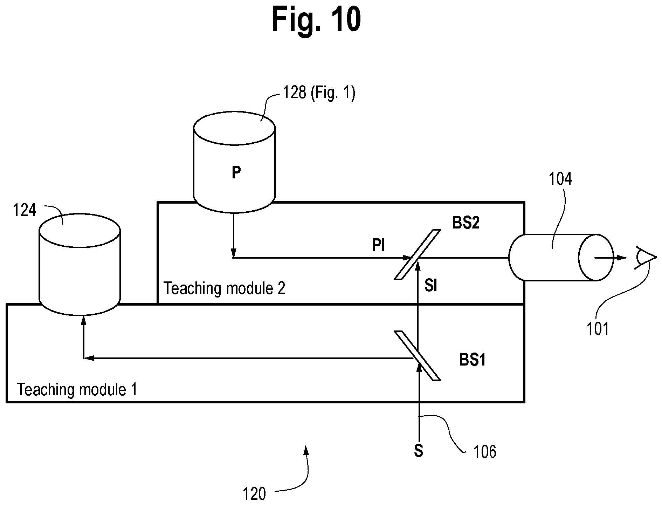

The augmented microscope, shown in Figure 1, includes a commercial off-the-shelf brightfield microscope (Nikon Eclipse Ni-U) with custom modules designed to capture high resolution images and superimpose the digital display content, free of parallax. Referring now to Figure 10, the standard upright microscope illuminates the specimen (S) from behind and captures the image rays with a conventional objective. These rays 106 propagate upward, in a collimated state, towards the oculars. A teaching module (Nikon Y-IDP) with a beam splitter (BS1) was inserted into the optical pathway in the collimated light space. This module was modified to accept a microscope camera (124), so that the specimen image relayed from BS1 was in focus at the camera sensor when the specimen was also in focus to the observer 101. A second customized teaching module (Nikon T-THM) was inserted between the oculars and the first teaching module. The beam splitter in this module (BS2) was rotated 90 degrees to combine light from the specimen image (SI) with that from the projected image (PI). Collimating optics between the microdisplay 128 (Figure 1) and BS2 were chosen to match the display size with the ocular size (22 mm). A relay optic was adjusted such that the microdisplay was positioned in a virtual focal plane of the specimen. In this way the viewer observes SI and PI in the same plane, which minimizes relative motion when the observer moves, a phenomenon known as parallax. Note that BS1 needs to be before BS2 in the optical pathway from objective to ocular, so that camera 124 sees a view of the specimen without the projection PI. The observer 101 looking through the eyepiece 104 sees PI superimposed onto SI.

Component design and selection was driven by final performance requirements. Camera and display devices were chosen for effective cell and gland level feature representation. The camera 124 (Adimec S25A80) included a 5120×5120 pixel color sensor with high sensitivity and global shutter capable of running up to 80 frames/sec. Camera images were captured by an industrial frame-grabber board (Cyton CXP-4) with PCI-E interface to the workstation. The microdisplay (eMagin SXGA096, 1292 × 1036 pixels) was mounted on the side of the microscope and imaged with an achromatic condenser (Nikon MBL71305) at a location tuned to minimize parallax and ensure that the specimen and display image are simultaneously in focus. The microdisplay includes an HDMI interface for receiving images from the workstation. Due to the limited brightness of this display, BS2 was chosen to transmit 90% of the light from the display and 10% from the sample, which resulted in adequate contrast between PI and SI when operating the microscope light source near half of its maximum intensity.

Software and Hardware System

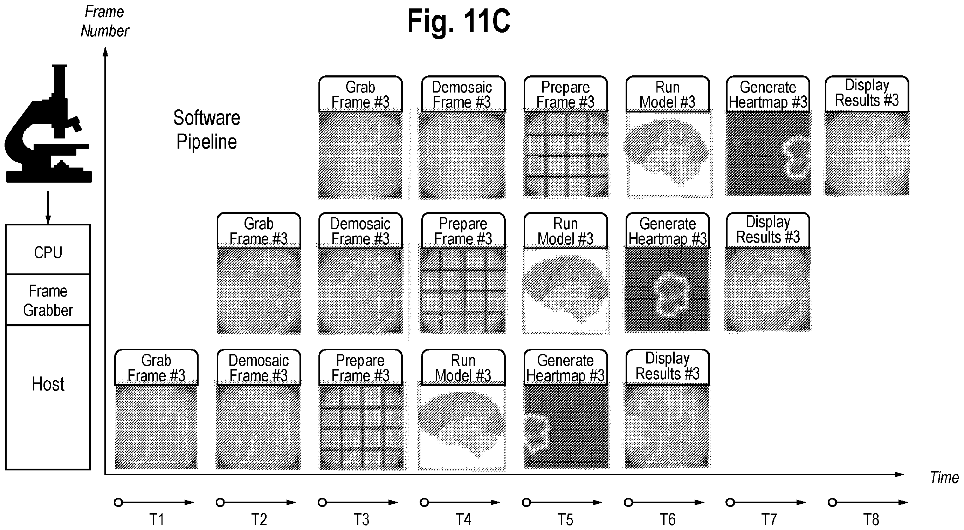

The application driving the entire system runs on a standard off-the-shelf PC with a BitFlow frame grabber connected to a camera 124 (Fig. 1, 10)) for live image capture and an NVidia Titan Xp GPU for running deep learning algorithm. The end-to-end process from frame grabbing to the final display is shown in Figure 11C. To improve responsiveness, the system is implemented as a highly optimized, pipelined, multi-threaded process, resulting in low overall latency. The software is written in C++ and TensorFlow.

The primary pipeline consists of a set of threads that continuously grab an image frame from the camera, debayer it (i.e. convert the raw sensor output into a color image), prepare the data, run algorithm, process the results, and finally display the output. Other preprocessing steps such as flat-field correction and white balancing can be done in this thread as well for cameras which cannot do them directly on-chip. To reduce the overall latency, these steps run in parallel for a sequence of successive frames, i.e. the display of frame 'N', generation of heatmap of frame 'N+1', and running algorithm on frame 'N+2' all happen in parallel.

In addition to this primary pipeline, the system also runs a background control thread One purpose of this thread is to determine whether the camera image is sufficiently in focus to yield accurate deep learning algorithm results. The system uses an out-of-focus detection algorithm to assess focus quality. A second purpose of this thread is to determine the currently used microscope objective, so that the deep learning algorithm tuned for the respective magnification is used. Additionally, settings for white balance and exposure time on camera 124 can be set to optimal profiles for the respective lens.

To enable AI algorithms on analog microscope requires three novel technologies working in unison. First, state-of-the-art convolutional neural networks for high accuracy detection and classification are meeded. High accuracy neural networks have been shown possible in the literature on digitally scanned images alone or images from microscope alone. A contribution of this work is the demonstration of successful generalization of deep learning algorithms from digitally scanned images to microscope images. The feasibility of overcoming the differences in image modality allows us to use digitally scanned images for deep learning algorithm development for the ARM. Second, the ability to run these algorithms in real-time to provide an interactive user experience is needed. This is achieved by a tightly integrated software, hardware and AI algorithm system with a real-time performance for live algorithm predictions. Third, a parallax-free head-up display in the microscope to project high-resolution heatmaps, contours, or textual information onto the sample is needed. This is made possible by the novel configuration of the optic components that are generally available.

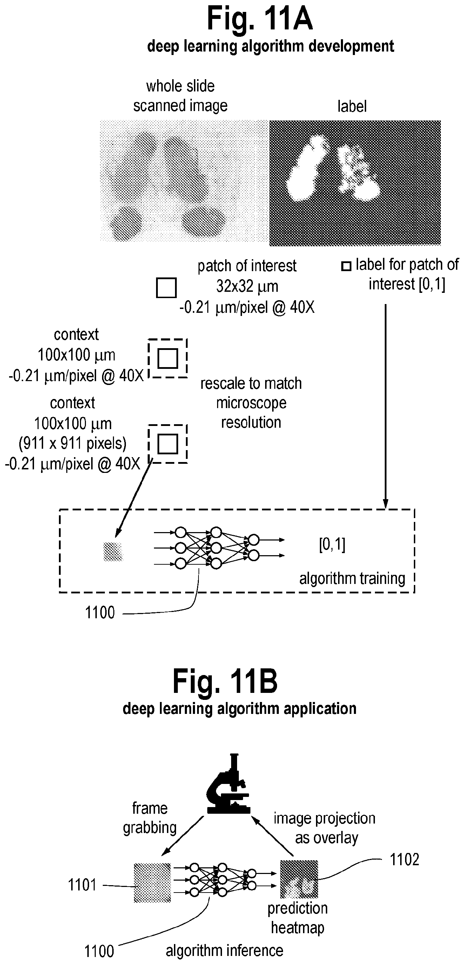

The deep learning image analysis workflow includes two phases: algorithm development and algorithm application, illustrated in Figure 11A and 11B, respectively. For algorithm development, Figure 11A, we train the neural networks 1100 on digitized pathology slides with patches of size $911\times911$ pixels at each of the magnifications (4X, 10X, 20X, 40X for lymph node and 4X, 10X, 20X for prostate), and the corresponding labels indicating the diagnosis, e.g. tumor/benign or Gleason grades. By changing the weights of the neural network to reduce the difference between the predicted results and the corresponding labels, the neural network learned to recognize patterns and distinguish between different diagnoses. During neural network training, we also scaled the whole slide images to match the pixel resolution from the scanner images (~0.21 μm/pixel) to the pixel resolution from the microscope camera (~0.11 μm/pixel). Although pixel scaling can be done in real-time on the ARM, we chose to do it in the neural network training step to shift the required computation into the training phase.

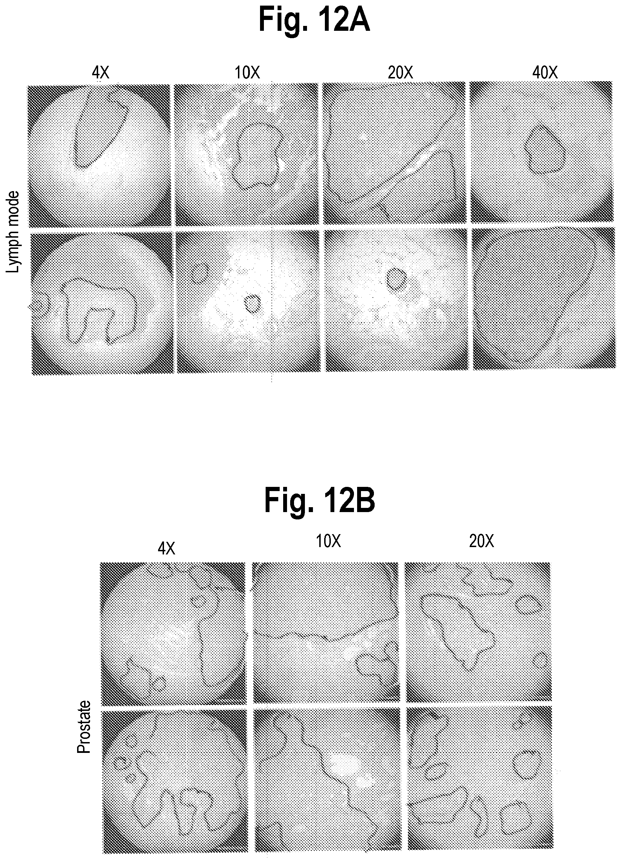

For algorithm application, Figure 11B, the neural network 1100 is provided with images 1101 of size $5120\times5120$ pixels captured from the microscope camera. The output from the network is a heatmap 1102 depicting the likelihood of cancer at each pixel location. The heatmap can be displayed directly using a color map, or thresholded to get an outline that is then displayed as an overlay (P1, Figure 10) on the sample. Microscope users found that outlines are usually favored over heatmaps in these applications since the latter occludes the underlying sample. However, heatmaps could be useful in some applications, and the system is capable of displaying either visualization mode. Examples of outlines generated and superimposed on the field of view in the eyepiece showing areas of cancer cells in lymph node tissue are shown in Figure 12A at 4X, 10X, 20X and 40X, in prostate tissue shown in Figure 12B at the same magnification levels.

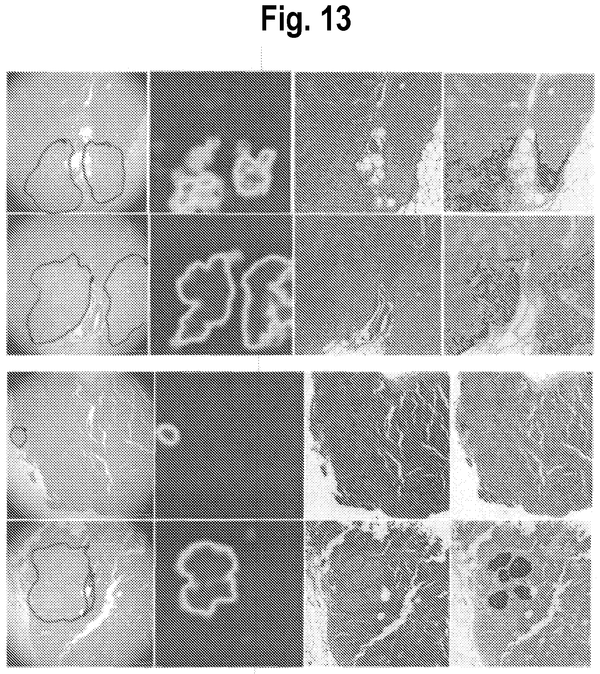

Figure 13 shows several sample fields of view of lymph node specimens for metastasis detection, the first column being the augmented reality image with the overlay in the form of an outline, the second column being the heat map generated by the neural network, the third column showing the whole slide H&E image and the fourth column showing the whole slide IHC image. To validate the algorithm performance, we also show the digitally scanned H&E images and IHC images on the side for comparison. Despite the differences in image quality and color distribution between microscope images and digitally scanned images, our algorithms correctly identify the tumor region in the fields of view.

Figure 14A-F are illustrations of examples of fields of view of microscope with an overlay or enhancement in the form of quantitative measurements. Figure 14A shows a tissue sample with a border and a measurement of "100% PR" meaning all the cells within the border are positive for expression of the progesterone receptor protein. Figure 14B shows an enhancement in the form of circles drawn around cells undergoing mitosis and a quantitative report of the number of cells per high power field: "4 mitoses per high power field." Figure 14C shows a measurement or ruler showing the dimension (200 μm) of a cluster of cells. Figure 14D shows a biological sample with dark points indicating areas where the machine learning model identified the presence of individual microorganisms, in this case heliobater pylori.

Algorithm Evaluation

We evaluate the algorithm performance of tumor detection within the field of view with the following metrics: receiver operating characteristic (ROC) curves (the true positive rate against the false positive rate), area under the ROC curve (AUC), accuracy, precision, and recall (TP: true positive; FP: false positive; FN: false negative):

$$Precision = TP/(TP+FP),$$

$$Recall/True\ Positive\ Rate = TP/(TP+FN),$$

$$False\ Positive\ Rate = FP/(FP+TN).$$

The following performance metrics were observed:

| Tissue Type | Operating Threshold | Accuracy ($Cl_{95}$) | Precision ($Cl_{95}$) | Recall ($Cl_{95}$) |
|---|---|---|---|---|
| lymph node | High Accuracy | **0.96 (0.92-0.99)** | 0.98 (0.53-1.00) | 0,94 (0.86-1.00) |
| | High Precision | 0.92 (0.86-0.97) | **0.98 (0.92-1.00)** | 0.85 (0.75-0.85) |
| | High Recall | 0.89 (0.81-0.95) | 0.82 (0.72-0.92) | **0.98 (0.93-1.00)** |
| prostate | High Accuracy | **0.96 (0.92-0.99)** | 0.94 (0.87-1.00) | 0.98 (0.93-1.00) |
| | High Precision | 0.95 (0.90-0.99) | **0.96 (0.89-1.00)** | 0.94 (0.86-1.00) |
| | High Recall | 0.96 (0.92-0.99) | 0.94 (0.87-1.00) | **0.98 (0.93-108)** |

Receiver operating characteristic (ROC) plots are provided in the appendix of the U,S, Provisional application cited at the beginning of this document.

Results

Using modern deep learning algorithms with off-the-shelf graphics card for accelerated computation, the system achieved a total latency of about 100 ms (10 frames per second) which is fast enough for most workflows. The projection of the deep learning predictions into the optics was high enough contrast to be clearly visible on top of the tissue sample using common background illumination levels and was parallax-free. Operating the ARM was seamless for first-time users (pathologists that were not part of the study) who tested it, with almost no learning curve

User Interface for set-up and configuration

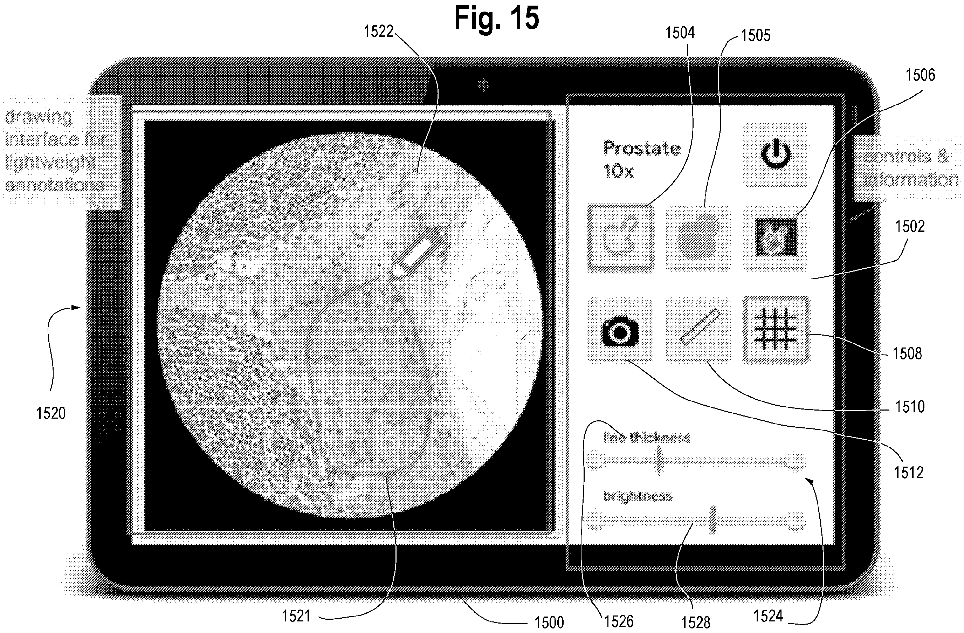

In one possible configuration the workstation 140 (Figure 1), e.g., a computing device such as a laptop or tablet computer) associated with the microscope may include a graphical display which provides tools to allow the user to configure various parameters for display of the enhancement in the field of view. For example, as shown in Figure 15, a tablet or workstation display 1500 includes a controls and information pane which includes various icons which when activated provide a menu of options to configure various features of the overlay. The icon 1504 provides a menu of options to control the color or darkness of borders that are superimposed in the field of view. The icon 1505 provides a menu of options to control the display of area calculations and shading for areas in the image, such as cells or clusters of cells. The icon 1506 provides a menu of options to configure the display of "heat maps", for example colored pixel regions that indicate the likelihood of image data containing cancer cells. The icon 1508 leads to a menu of options for configuring grids superimposed on the image, such grids being used for cell counting or other counting operations within a given region defined by an element in the grid. Such grid counts can be used to extrapolate to density calculations for an overall slide or for some portion thereof. The icon 1510 leads to a menu of options for configuring measuring tools and labelling of measurements on the display as an overlay, such as shown in Figure 14C.

The pane 1520 on the left side of the display 1500 provides a drawing interface for the user to manually create annotations on the displayed image, for example by means of a finger stroke on a touch sensitive display or by mouse action on a conventional display. The image 1522 displayed is the current field of view of the microscope and the user is provided with the ability to draw an outline over regions of interest (as shown). The outline is preserved along with the image of the field of view. The region 1524 of the display provides drawing tools to control the user annotations, including a scroll bar 1526 to change line thickness and a scroll bar 1528 to change the brightness of the line.

It is further contemplated that configuration of the annotations or the AR display superimposed on the field of view can be done by voice command, e.g., using the workstation 140 associated with the AR microscope as in Figure 1.

Quantitative analysis and enhancements

Quantitative measurements of human epidermal growth factor receptor 2 (HER2), Estrogen Receptor (ER), and Progesterone Receptor (PR) immunohistochemistry (IHC) biomarker expression is critical for breast cancer therapy selection. These advances in cancer classification, treatment and associated companion diagnostics have driven increasing complexity and reporting requirements for the pathologist, in some cases stretching human capability for efficient assessment.

Even though this study details two clinical diagnostic applications, the AR microscope of this disclosure is application agnostic and can be used for any kind of image analysis task for which a deep learning algorithm has been trained. We envision the usage of this device for many other clinical and research applications, including quantitative analysis, such as mitotic rate estimation, IHC quantification and positive margin detection in frozen sections. Figures 14A-14F shows several images captured from the microscope with examples of suitable information overlays including biomarker/IHC quantification (HER2, ER, PR), with Figure 14A showing PR quantitation (with the overlay identifying a group of cells with 100 % cells having PR gene expression), mitotic figure counting (Figure 14B, with the overlay being identification of cells currently undergoing mitosis and a measure of the number of such cells per high power field); measurement of tumor size (Figure 14C, overlay being a measurement index or ruler showing the size of a tumor or cluster of tumor cells); identification of infectious disease agents, such as Heliobacter pylori (Figure 14D, overlay being colored points or circles of individual bacteria or clusters of bacteria); identification of Mycobacterium (Figure 14E, overlay in the form of a circle or region highlighting a Mycobacterium), and identification of prostate cancer region with estimation of percentage of tumor involvement (Figure 14F, overlay in the form of regions predicted positive for prostate cancer and a percentage of the specimen having tumor involvement) . Other applications include translational experiments that explore the quantification of immune cells, immunotherapy biomarkers such as PDL1, and cell cycle markers such as P53 and Ki67 using IHC. Additional applications include the analysis of fluorescent (FISH) images, live cell culture images, and even applications outside of life sciences such as in semiconductor quality control.

All the measurements that are made in a quantitative annotations can be saved as structured metadata (e.g., .xml files) along with the field of view image captured by the AR microscope camera of Figure 1.

All measurements that are taken per field of view can be aggregated (e.g., summed or averaged) across multiple fields of view, for instance to estimate the overall density of tumor cells or other features. This could also include tracking the features in the field of view, and thereby determining whether the current field of view has been analyzed already. This tracking enables several other possible features: 1) the ability to show cached results that were previously computed; 2) avoid double counting the area in the field of view, and 3) combine or "stitch" the measurements/metrics across the slide to obtain for example a density map that is larger than single fields of view. In this regard, a low power image of the slide may be obtained initially and used to create essentially a map of the slide; this map (and the pixel or stage coordinates associated with the map) can be used to keep track of where in the map the current field of view is. Feature tracking techniques may also be used to know where in the overall slide the current field of view is positioned. Some microscopes include motorized stages and a hardware solution for keeping track of the position of the microscope objective/field of view relative to the overall slide and this solution, or a lower cost measuring version available on less complex microscopes can be used for creating the map and tracking the position of the field of view.

In quantitative analysis, some measurements do not require sampling or counting the entire slide, but rather they employ a technique where only a certain number of fields of view need to be measured. For example some measurements require say 5 or 10 fields of view at high power, and the results are aggregated or averaged over such fields of view.

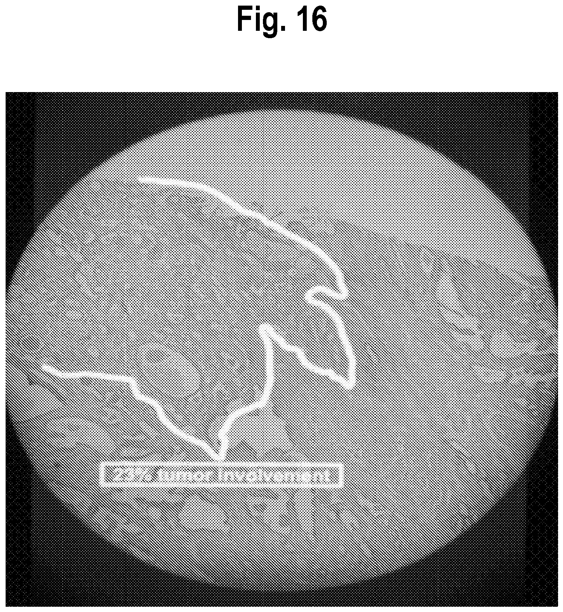

Figure 16 is an example of an annotation superimposed on the field of view in the form of an outline surrounding areas determined by the machine learning model to be cancer/tumor cells, and a text block providing a quantitative result: "23 % tumor involvement." The tumor involvement measurement, i.e., the percentage of the area within the outline having tumor cells, and optionally the area of tumor per tumor grade, is an important metric in diagnosis and prognosis. For example, in prostate cancer, Gleason grading also requires quantification of the Gleason 3 v. Gleason 3 v. Gleason 5 area. By using machine learning models which are trained to recognize tumors cells with particular Gleason numbers it is possible to provide these statistics as an overlay on the image. The area of interest (e.g., tumor) could be measured based on what the model detects in the image. Relative or absolute numbers could be displayed as an enhancement or overlay in the field of view. As another example, histograms (e.g., relative area of Gleason, 2, 3, 4, 5 etc. tumor regions) could also be displayed.

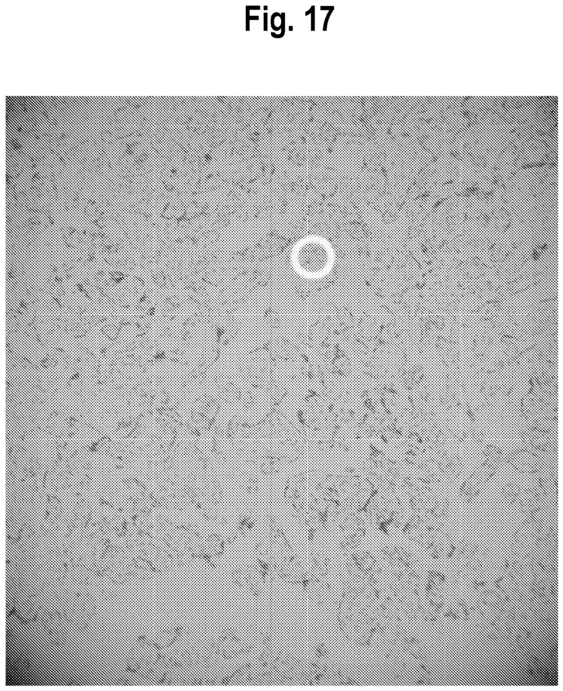

Figure 17 is an example of an overlay in the form of a circle indicating the detection of a mycobacterium, e.g., tuberculosis) by a machine learning model in the field of view. For some infectious diseases, classification and speciation is also important, such as Malaria. For those applications, the predicted species could also be displayed, including the likelihood of the respective class. For example, next to the circle could be data such as "77% Species A. 22% Species B, 1% other", indicating that the circled organism is most likely Species A. Figure 17 is also an illustration of the ability of the AR microscope to detect small objects, e.g., individual organism cells, at a scale that a human can detect at. The models may also be trained to detect objects at a smaller scale than humans can normally do so, for example at 40X when humans use 80X for the same task. Currently, oil immersion techniques are used for finding objects at very high power, such as 80X or 100X, and the present AR microscope may avoid the need for oil immersion methods. Inception deep neural network models can be developed to work at this scale.

Figure 14B is an example of detection of rare events and counting, in this example individual cells undergoing mitosis within a given high power magnification field of view. Such cells identified in the image by the machine learning model are annotated with a circle as shown in Figure 14B with quantitative values below, in this example "4 mitoses per high power field." Mitoses counting is done for breast cancer Nottingham grading. In routine clinical practice the number of mitoses is counted in a few fields of view and then extrapolated to the entire image. Display options include the absolute number or the density, as shown in Figure 14.

Figure 14C is an example of annotations in the form of length measurements. A collection of tumor cells identified by the machine learning model is then measured and the measurement reported as an annotation, in this case 200 μm and a measuring stick showing the points in the image from which the measurement was taken. Length measurements are an important detail that is reported for some diagnoses. For example, length or size measurements make the difference between micrometastasis versus macrometastasis in metastatic breast cancer, and can lead to different treatment pathways. Spatial extent of a feature of interest (e.g., metastasis) can be measured and displayed as shown in Figure 14C. The measurements can be linear (e.g., the largest dimension, as shown in Figure 14C) or can be curved, for example following the object boundary. Distance or scale can be derived from knowledge of the microscope objective used to capture the image and camera details such as the pixel size and pitch.

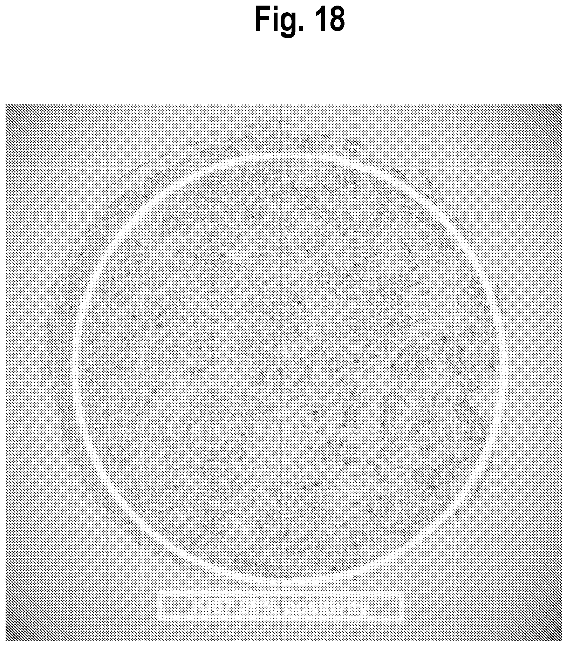

Figure 18 is an image of a field of view obtained by the camera in the microscope along with the display of an overlay in the form of biomarker quantitation, in this example the number of cells positive for expression of the protein Ki67 (98% in this example). To generate such statistics, two machine learning models are used which process the image data: 1) a machine learning model trained to detect individual cells, e.g., by finding individual cell nuclei, and counting all of such cells in the image; and 2) a machine learning model trained to detect cells expressing Ki-67, and counting such cells. In IHC applications such as this the slide is typically stained with a staining agent which binds to the Ki-67 protein and causes positive cells to appear in a contrasting color; the machine learning model is trained to identify the cells positive for Ki-67 by identifying cells having this color signature. The statistics of the percent positive is arrived at by simply dividing the number of cells identified as positive for KI-67 in the field of view by the total number of cells in the field of view.

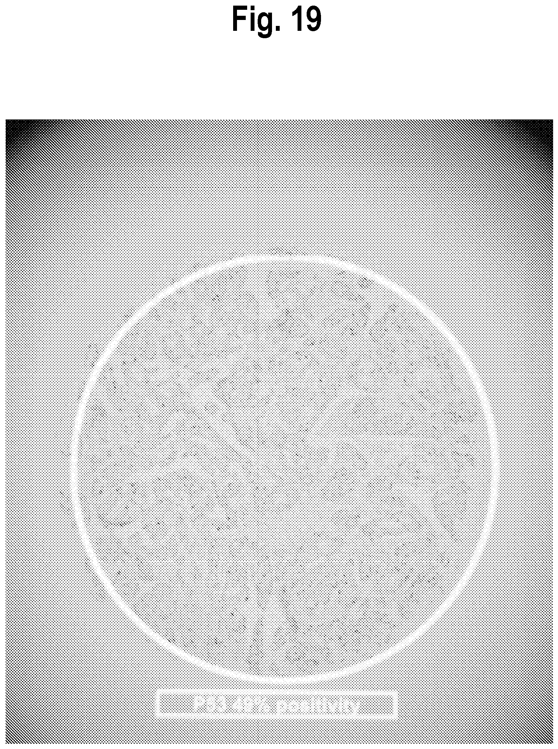

Figure 19 is another example of is an image of a field of view obtained by the camera in the microscope along with the display of an overlay in the form of biomarker quantitation, in this example the number of cells positive for expression of the protein P53 (49% in this example). To generate such statistics, two machine learning models are used which process the image data: 1) a machine learning model trained to detect individual cells, e.g., by finding individual cell nuclei, and counting all of such cells in the image: and 2) a machine learning model trained to detect cells expressing P53, and counting such cells. In IHC applications such as this the slide is also stained with a staining agent which binds to the P53 protein and causes such protein to appear in a contrasting color; the machine learning model is trained to identify the cells positive for P53 by identifying cells having this color signature. The statistics of the percent positive is arrived at by simply dividing the number of cells identified as positive for P53 by the total number of cells in the field of view.

While presently preferred embodiments are described with particularity, all questions concerning scope of the invention are to be answered by reference to the appended claims interpreted in light of the foregoing.

## Claims

1. A method for assisting a user in review of a slide (114) containing a biological sample with a microscope (100) having an eyepiece (104) comprising the steps of:

   (a) capturing, with a camera (124), a digital image of a view of the sample as seen through the eyepiece of the microscope,
   (b) using a first machine learning pattern recognizer (200) to identify one or more areas of interest in the sample from the image captured by the camera, and a second machine pattern recognizer trained to identify individual cells and

(c) superimposing an enhancement to the view of the sample as seen through the eyepiece of the microscope as an overlay, wherein the enhancement is based upon the identified areas of interest in the sample and further comprises quantitative data associated with the areas of interest,

(d) wherein, when the sample is moved relative to the microscope optics or when a magnification or focus of the microscope changes, a new digital image of a new view of the sample is captured by the camera and supplied to the machine learning pattern recognizer, and a new enhancement is superimposed onto the new view of the sample as seen through the eyepiece in substantial real time.

2. The method of claim 1, wherein the one or more areas of interest comprise cells positive for expression of a protein and wherein the quantitative data comprises a percent of the cells in the view as being positive for such protein expression.

3. The method of claim 2, wherein the protein comprises Ki-67, P53, Estrogen Receptor (ER) or Progesterone Receptor (PR).

4. The method of claim 1, wherein the one or more areas of interest comprise individual microorganism cells and the quantitative data comprises a count of the number of microorganism cells in the view.

5. The method of claim 1, wherein the one or more areas of interest comprise individual cells undergoing mitosis and wherein the quantitative data comprises a count of the number of cells in the view undergoing mitosis.

6. The method of claim 1, wherein the areas of interest comprise tumor cells and wherein the quantitative data comprises an area measurement of the tumor cells, either absolute or relative area within a defined region in the sample.

7. The method of any of claims 1-6, further comprising the step of providing on a workstation associated with the microscope a graphical display providing access to tools to customize the presentation of the enhancement on the field of view.

8. The method of claim 1, wherein the quantitative data comprises a measurement.

9. The method of claim 8, wherein the measurement comprises an area measurement and wherein the areas of interest comprise prostate tissue with specific Gleason grades.

10. The method of claim 1, wherein the quantitative data comprises a count of the number of areas of interest in the view.

11. A system (100) assisting a user in review of a slide containing a biological sample comprising:

a microscope having a stage (110) for holding a slide (114) containing a biological sample, at least one objective lens (108), and an eyepieces (104),
a digital camera (124) configured to capture digital images of a view of the sample as seen through the eyepiece of the microscope,
a compute unit (126) comprising a machine learning pattern recognizer (200) configured to receive the digital images from the digital camera, wherein the pattern recognizer is trained to identify regions of interest in biological samples of the type currently placed on the stage, and wherein the pattern recognizer recognizes regions of interest on a digital image captured by the camera and wherein the compute unit generates data representing an enhancement to the view of the sample as seen through the eyepiece of the microscope, wherein the enhancement is based upon the regions of interest in the sample; and
one or more optical components (120, 122, 128, 130) coupled to the eyepiece for superimposing the enhancement on the field of view;
wherein the compute unit implements a first machine learning pattern recognizer trained to identify individual cells within the view and a second machine learning pattern recognizer trained to identify individual cells within the view which are positive for expression of a protein.
and wherein the enhancement further comprises a display of quantitative data relating to the cells which are positive for the expression of the protein.

12. The system of claim 10, wherein the protein comprises Ki-67, P53, Estrogen Receptor or Progesterone Receptor (PR).

**13.** A system (100) assisting a user in review of a slide containing a biological sample comprising:

a microscope having a stage (110) for holding a slide (114) containing a biological sample, at least one objective lens (108), and an eyepieces (104),

a digital camera (124) configured to capture digital images of a view of the sample as seen through the eyepiece of the microscope,

a compute unit (126) comprising a machine learning pattern recognizer (200) configured to receive the digital images from the digital camera, wherein the pattern recognizer is trained to identify regions of interest in biological samples of the type currently placed on the stage, and wherein the pattern recognizer recognizes regions of interest on a digital image captured by the camera and wherein the compute unit generates data representing an enhancement to the view of the sample as seen through the eyepiece of the microscope, wherein the enhancement is based upon the regions of interest in the sample; and

one or more optical components (120, 122, 128, 130) coupled to the eyepiece for superimposing the enhancement on the field of view;

wherein the compute unit implements a machine learning pattern recognizer trained to identify individual cells which are undergoing mitosis;

and wherein the enhancement further comprises a display of quantitative data relating to the cells which are undergoing mitosis.

**14.** A system (100) assisting a user in review of a slide containing a biological sample comprising:

a microscope having a stage (110) for holding a slide (114) containing a biological sample, at least one objective lens (108), and an eyepieces (104),

a digital camera (124) configured to capture digital images of a view of the sample as seen through the eyepiece of the microscope,

a compute unit (128) comprising a machine learning pattern recognizer (200) configured to receive the digital images from the digital camera, wherein the pattern recognizer is trained to identify regions of interest in biological samples of the type currently placed on the stage, and wherein the pattern recognizer recognizes regions of interest on a digital image captured by the camera and wherein the compute unit generates data representing an enhancement to the view of the sample as seen through the eyepiece of the microscope, wherein the enhancement is based upon the regions of interest in the sample; and

one or more optical components (120, 122, 128, 130) coupled to the eyepiece for superimposing the enhancement on the field of view;

wherein the compute unit implements one or more machine learning pattern recognizers trained to identify individual tumor cells or areas of tumor cells which are classified in accordance with specific Gleason grades, and wherein the enhancement further comprises a display of quantitative area data relating to the tumor cells or areas of tumor cells which are classified in accordance with specific Gleason grades.

**15.** The system of any of claims 11-14, further comprising a workstation (140) associated with the microscope having a display;

wherein the display providing tools for a user of the workstation to draw an annotation on an image of the view, and wherein the annotation is saved along with the image of the view in a computer memory; and/or

wherein the display providing access to tools to customize the presentation of the enhancement on the field of view.

**Patentansprüche**

**1.** Verfahren zum Unterstützen eines Benutzers bei der Überprüfung eines Objektträgers (114), der eine biologische Probe enthält, mit einem Mikroskop (100), das über ein Okular (104) verfügt, die folgenden Schritte umfassend:

(a) Erfassen, mit einer Kamera (124), eines Digitalbilds einer Ansicht der Probe, wie durch das Okular des Mikroskops gesehen,

(b) Verwenden einer ersten Maschinenlernen-Mustererkennungsvorrichtung (200) zum Identifizieren eines oder mehrerer Bereiche von Interesse in der Probe aus dem durch die Kamera erfassten Bild und einer zweiten Maschinenlernen-Mustererkennungsvorrichtung, die zum Identifizieren einzelne Zellen trainiert ist, und

(c) Überlagern einer Verbesserung der Ansicht der Probe, wie durch das Okular des Mikroskops gesehen, als eine Überlagerung, wobei die Verbesserung auf den identifizierten Bereichen von Interesse in der Probe basiert und ferner quantitative Daten, die mit den Bereichen von Interesse assoziiert sind, umfasst,

(d) wobei, wenn die Probe relativ zur Optik des Mikroskops bewegt wird oder wenn sich eine Vergrößerung oder eine Scharfeinstellung des Mikroskops verändert, ein neues Digitalbild einer neuen Ansicht der Probe durch die Kamera erfasst wird und der Maschinenlernen-Mustererkennungsvorrichtung zugeführt wird und eine neue Verbesserung der neuen Ansicht der Probe, wie durch das Okular gesehen, in im Wesentlichen Echtzeit überlagert wird.

2. Verfahren nach Anspruch 1, wobei der eine oder die mehreren Bereiche von Interesse Zellen umfassen, die für die Expression eines Proteins positiv sind, und wobei die quantitativen Daten einen Prozentanteil der Zellen in der Ansicht umfassen, die für eine derartige Proteinexpression positiv sind.

3. Verfahren nach Anspruch 2, wobei das Protein Ki-67, P53, Östrogenrezeptor (ER) oder Progesteronrezeptor (PR) umfasst.

4. Verfahren nach Anspruch 1, wobei der eine oder die mehreren Bereiche von Interesse einzelne Mikroorganismuszellen umfassen und die quantitativen Daten eine Zählung der Anzahl von Mikroorganismuszellen in der Ansicht umfassen.

5. Verfahren nach Anspruch 1, wobei der eine oder die mehreren Bereiche von Interesse einzelne Zellen, die eine Mitose durchlaufen, umfassen und wobei die quantitativen Daten eine Zählung der Anzahl von Zellen in der Ansicht, die eine Mitose durchlaufen, umfasst.

6. Verfahren nach Anspruch 1, wobei die Bereiche von Interesse Tumorzellen umfassen und wobei die quantitativen Daten eine Bereichsmessung der Tumorzellen, entweder eines absoluten oder eines relativen Bereichs innerhalb einer definierten Region in der Probe, umfassen.

7. Verfahren nach einem der Ansprüche 1-6, ferner umfassend den Schritt des Bereitstellens, auf einer mit dem Mikroskop assoziierten Arbeitsstation, einer grafischen Anzeige, die Zugang zu Werkzeugen zum Anpassen der Präsentation der Verbesserung auf dem Sichtfeld bereitstellt.

8. Verfahren nach Anspruch 1, wobei die quantitativen Daten eine Messung umfassen.

9. Verfahren nach Anspruch 8, wobei die Messung eine Bereichsmessung umfasst und wobei die Bereiche von Interesse Prostatagewebe mit spezifischen Gleason-Graden umfassen.

10. Verfahren nach Anspruch 1, wobei die quantitativen Daten eine Zählung der Anzahl von Bereichen von Interesse in der Ansicht umfassen.

11. System (100), das einen Benutzer bei der Überprüfung eines Objektträgers, der eine biologische Probe enthält, unterstützt, umfassend:

ein Mikroskop mit einem Objekttisch (110) zum Halten eines Objektträgers (114), der eine biologische Probe enthält, mindestens einer Objektivlinse (108) und einem Okular (104), einer Digitalkamera (124), konfiguriert zum Erfassen von Digitalbildern einer Ansicht der Probe, wie durch das Okular des Mikroskops gesehen, eine Recheneinheit (126), umfassend eine Maschinenlernen-Mustererkennungsvorrichtung (200), konfiguriert zum Empfangen der Digitalbilder von der Digitalkamera, wobei die Mustererkennungsvorrichtung zum Identifizieren von Regionen von Interesse in biologischen Proben des Typs, der gegenwärtig auf dem Objekttisch platziert ist, trainiert ist und wobei die Mustererkennungsvorrichtung Regionen von Interesse auf einem durch die Kamera erfassten Digitalbild erkennt und wobei die Recheneinheit Daten erzeugt, die eine Verbesserung der Ansicht der Probe, wie durch das Okular des Mikroskops gesehen, repräsentieren, wobei die Verbesserung auf den Regionen von Interesse in der Probe basiert; und eine oder mehrere optische Komponenten (120, 122, 128, 130), die an das Okular zum Überlagern der Verbesserung auf dem Sichtfeld gekoppelt sind; wobei die Recheneinheit eine erste Maschinenlernen-Mustererkennungsvorrichtung, die zum Identifizieren einzelner Zellen innerhalb der Ansicht trainiert ist, und eine zweite Maschinenlernen-Mustererkennungsvorrichtung, die zum Identifizieren einzelner Zellen innerhalb der Ansicht, die zur Expression eines Proteins positiv sind, trainiert ist, implementiert

und wobei die Verbesserung ferner eine Anzeige quantitativer Daten, die zu den Zellen, die für die Expression des Proteins positiv ist, in Beziehung stehen, umfasst.

12. System nach Anspruch 10, wobei das Protein Ki-67, P53, Östrogenrezeptor oder Progesteronrezeptor (PR) umfasst.

13. System (100), das einen Benutzer bei der Überprüfung eines Objektträgers, der eine biologische Probe enthält, unterstützt, umfassend:

ein Mikroskop mit einem Objekttisch (110) zum Halten eines Objektträgers (114), der eine biologische Probe enthält, mindestens einer Objektivlinse (108) und einem Okular (104), einer Digitalkamera (124), konfiguriert zum Erfassen von Digitalbildern einer Ansicht der Probe, wie durch das Okular des Mikroskops gesehen, eine Recheneinheit (126), umfassend eine Maschinenlernen-Mustererkennungsvorrichtung (200), konfiguriert zum Empfangen der Digitalbilder von der Digitalkamera, wobei die Mustererkennungsvorrichtung zum Identifizieren von Regionen von Interesse in biologischen Proben des Typs, der gegenwärtig auf dem Objekttisch platziert ist, trainiert ist und wobei die Mustererkennungsvorrichtung Regionen von Interesse auf einem durch die Kamera erfassten Digitalbild erkennt und wobei die Recheneinheit Daten erzeugt, die eine Verbesserung der Ansicht der Probe, wie durch das Okular des Mikroskops gesehen, repräsentieren, wobei die Verbesserung auf den Regionen von Interesse in der Probe basiert; und eine oder mehrere optische Komponenten (120, 122, 128, 130), die an das Okular zum Überlagern der Verbesserung auf dem Sichtfeld gekoppelt sind; wobei die Recheneinheit eine Maschinenlernen-Mustererkennungsvorrichtung implementiert, die zum Identifizieren einzelner Zellen, die eine Mitose durchlaufen, trainiert ist; und wobei die Verbesserung ferner eine Anzeige quantitativer Daten, die zu den Zellen, die eine Mitose durchlaufen, in Beziehung stehen, umfasst.

14. System (100), das einen Benutzer bei der Überprüfung eines Objektträgers, der eine biologische Probe enthält, unterstützt, umfassend:

ein Mikroskop mit einem Objekttisch (110) zum Halten eines Objektträgers (114), der eine biologische Probe enthält, mindestens einer Objektivlinse (108) und einem Okular (104), einer Digitalkamera (124), konfiguriert zum Erfassen von Digitalbildern einer Ansicht der Probe, wie durch das Okular des Mikroskops gesehen, eine Recheneinheit (128), umfassend eine Maschinenlernen-Mustererkennungsvorrichtung (200), konfiguriert zum Empfangen der Digitalbilder von der Digitalkamera, wobei die Mustererkennungsvorrichtung zum Identifizieren von Regionen von Interesse in biologischen Proben des Typs, der gegenwärtig auf dem Objekttisch platziert ist, trainiert ist und wobei die Mustererkennungsvorrichtung Regionen von Interesse auf einem durch die Kamera erfassten Digitalbild erkennt und wobei die Recheneinheit Daten erzeugt, die eine Verbesserung der Ansicht der Probe, wie durch das Okular des Mikroskops gesehen, repräsentieren, wobei die Verbesserung auf den Regionen von Interesse in der Probe basiert; und eine oder mehrere optische Komponenten (120, 122, 128, 130), die an das Okular zum Überlagern der Verbesserung auf dem Sichtfeld gekoppelt sind; wobei die Recheneinheit eine oder mehrere Maschinenlernen-Mustererkennungsvorrichtungen implementiert, die zum Identifizieren einzelner Tumorzellen oder von Bereichen von Tumorzellen, die gemäß spezifischen Gleason-Graden klassifiziert sind, trainiert sind, und wobei die Verbesserung ferner eine Anzeige von quantitativen Bereichsdaten, die zu den Tumorzellen oder Bereichen von Tumorzellen, die gemäß spezifischen Gleason-Graden klassifiziert sind, in Beziehung stehen, umfasst.

15. System nach einem der Ansprüche 11-14, ferner umfassend eine mit dem Mikroskop assoziierte Arbeitsstation (140) mit einer Anzeige;

wobei die Anzeige Werkzeuge für einen Benutzer der Arbeitsstation zum Zeichnen einer Anmerkung auf dem Bild der Anzeige bereitstellt und wobei die Anmerkung zusammen mit dem Bild der Ansicht in einem Computerspeicher gespeichert wird; und/oder wobei die Anzeige Zugang zu Werkzeugen zum Anpassen der Präsentation der Verbesserung auf dem Sichtfeld bereitstellt.

**Revendications**

1. Procédé pour assister un utilisateur dans l'examen d'une lame (114) contenant un échantillon biologique avec un microscope (100) possédant un oculaire (104) comprenant les étapes :

   (a) de capture, avec une caméra (124), d'une image numérique d'une vue de l'échantillon tel que vu à travers l'oculaire du microscope,
   (b) d'utilisation d'un premier dispositif de reconnaissance des formes par apprentissage machine (200) pour identifier une ou plusieurs zones d'intérêt dans l'échantillon à partir de l'image capturée par la caméra, et d'un second dispositif de reconnaissance des formes par apprentissage machine entraîné à identifier des cellules individuelles et
   (c) de superposition d'une amélioration de la vue de l'échantillon tel que vu à travers l'oculaire du microscope en tant que superposition, dans lequel l'amélioration s'effectue sur la base des zones d'intérêt identifiées dans l'échantillon et comprend en outre des données quantitatives associées aux zones d'intérêt,
   (d) dans lequel, lorsque l'échantillon est déplacé par rapport à l'optique du microscope ou lorsqu'un grandissement ou une mise au point du microscope change, une nouvelle image numérique d'une nouvelle vue de l'échantillon est capturée par la caméra et fournie au dispositif de reconnaissance des formes par apprentissage machine, et une nouvelle amélioration est superposée sur la nouvelle vue de l'échantillon tel que vu à travers l'oculaire sensiblement en temps quasi réel.

2. Procédé selon la revendication 1, dans lequel les une ou plusieurs zones d'intérêt comprennent des cellules positives pour l'expression d'une protéine et dans lequel les données quantitatives comprennent un pourcentage des cellules dans la vue comme étant positives pour une telle expression de protéine.

3. Procédé selon la revendication 2, dans lequel la protéine comprend Ki-67, P53, un récepteur d'oestrogène (ER) ou un récepteur de progestérone (PR).

4. Procédé selon la revendication 1, dans lequel les une ou plusieurs zones d'intérêt comprennent des cellules de micro-organismes individuelles et les données quantitatives comprennent un décompte du nombre de cellules de micro-organismes dans la vue.

5. Procédé selon la revendication 1, dans lequel les une ou plusieurs zones d'intérêt comprennent des cellules individuelles en cours de mitose et dans lequel les données quantitatives comprennent un décompte du nombre de cellules dans la vue en cours de mitose.

6. Procédé selon la revendication 1, dans lequel les zones d'intérêt comprennent des cellules tumorales et dans lequel les données quantitatives comprennent une mesure de zone des cellules tumorales, soit dans une zone absolue soit relative dans une région définie dans l'échantillon.

7. Procédé selon l'une quelconque des revendications 1 à 6, comprenant en outre l'étape de fourniture sur une station de travail associée au microscope d'un dispositif d'affichage graphique fournissant un accès aux outils pour personnaliser la présentation de l'amélioration sur le champ de vue.

8. Procédé selon la revendication 1, dans lequel les données quantitatives comprennent une mesure.

9. Procédé selon la revendication 8, dans lequel les mesures comprennent une mesure de zone et dans lequel les zones d'intérêt comprennent du tissu prostatique avec des scores de Gleason.

10. Procédé selon la revendication 1, dans lequel les données quantitatives comprennent un décompte du nombre de zones d'intérêt dans la vue.

11. Système (100) assistant un utilisateur dans l'examen d'une lame contenant un échantillon biologique, comprenant :

    un microscope possédant une platine (110) pour maintenir une lame (114) contenant un échantillon biologique, au moins une lentille d'objectif (108), et un oculaire (104), une caméra numérique (124) configurée pour capturer des images numériques d'une vue de l'échantillon tel que vu à travers l'oculaire du microscope,
    une unité de calcul (126) comprenant un dispositif de reconnaissance des formes par apprentissage machine (200) configurée pour recevoir les images numériques à partir de la caméra numérique, dans lequel le dispositif de reconnaissance des formes est entraîné à identifier des régions d'intérêt dans les échantillons biologiques du type actuellement placé sur la platine, et dans lequel le dispositif de reconnaissance des formes reconnaît des régions d'intérêt sur une image numérique capturée par la caméra et dans lequel l'unité de calcul génère des données représentant une amélioration de la vue de l'échantillon tel que vu à travers l'oculaire du microscope, dans lequel l'amélioration s'effectue sur la base des régions d'intérêt dans l'échantillon ; et

un ou plusieurs composants optiques (120, 122, 128, 130) couplés à l'oculaire pour superposer l'amélioration sur le champ de vue ;

dans lequel l'unité de calcul implémente un premier dispositif de reconnaissance des formes par apprentissage machine entraîné à identifier des cellules individuelles dans la vue et un second dispositif de reconnaissance des formes par apprentissage machine entraîné à identifier des cellules individuelles dans la vue qui sont positives pour l'expression d'une protéine.

et dans lequel l'amélioration comprend en outre un affichage de données quantitatives relatives aux cellules qui sont positives pour l'expression de la protéine.

12. Système selon la revendication 10, dans lequel la protéine comprend Ki-67, P53, un récepteur d'œstrogène ou un récepteur de progestérone (PR).

13. Système (100) assistant un utilisateur dans l'examen d'une lame contenant un échantillon biologique ; comprenant :

un microscope possédant une platine (110) pour maintenir une lame (114) contenant un échantillon biologique, au moins une lentille d'objectif(108), et un oculaire (104), une caméra numérique (124) configurée pour capturer des images numériques d'une vue de l'échantillon tel que vu à travers l'oculaire du microscope,

une unité de calcul (126) comprenant un dispositif de reconnaissance des formes par apprentissage machine (200) configurée pour recevoir les images numériques à partir de la caméra numérique, dans lequel le dispositif de reconnaissance des formes est entraîné à identifier des régions d'intérêt dans les échantillons biologiques du type actuellement placé sur la platine, et dans lequel le dispositif de reconnaissance des formes reconnaît des régions d'intérêt sur une image numérique capturée par la caméra et dans lequel l'unité de calcul génère des données représentant une amélioration de la vue de l'échantillon tel que vu à travers l'oculaire du microscope, dans lequel l'amélioration s'effectue sur la base des régions d'intérêt dans l'échantillon ; et

un ou plusieurs composants optiques (120, 122, 128, 130) couplés à l'oculaire pour superposer l'amélioration sur le champ de vue ;

dans lequel l'unité de calcul implémente un dispositif de reconnaissance des formes par apprentissage machine entraîné à identifier des cellules individuelles qui sont en cours de mitose ;

et dans lequel l'amélioration comprend en outre un affichage de données quantitatives relatives aux cellules qui sont en cours de mitose.

14. Système (100) assistant un utilisateur dans l'examen d'une lame contenant un échantillon biologique comprenant :

un microscope possédant une platine (110) pour maintenir une lame (114) contenant un échantillon biologique, au moins une lentille d'objectif(108), et un oculaire (104), une caméra numérique (124) configurée pour capturer des images numériques d'une vue de l'échantillon tel que vu à travers l'oculaire du microscope,

une unité de calcul (128) comprenant un dispositif de reconnaissance des formes par apprentissage machine (200) configurée pour recevoir les images numériques à partir de la caméra numérique, dans lequel le dispositif de reconnaissance des formes est entraîné à identifier des régions d'intérêt dans les échantillons biologiques du type actuellement placé sur la platine, et dans lequel le dispositif de reconnaissance des formes reconnaît des régions d'intérêt sur une image numérique capturée par la caméra et dans lequel l'unité de calcul génère des données représentant une amélioration de la vue de l'échantillon tel que vu à travers l'oculaire du microscope, dans lequel l'amélioration s'effectue sur la base des régions d'intérêt dans l'échantillon ; et

un ou plusieurs composants optiques (120, 122, 128, 130) couplés à l'oculaire pour superposer l'amélioration sur le champ de vue ;

dans lequel l'unité de calcul implémente un ou plusieurs dispositifs de reconnaissance des formes par apprentissage machine entraînés à identifier des cellules tumorales individuelles ou des zones de cellules tumorales qui sont classées par rapport à des scores de Gleason,

et dans lequel l'amélioration comprend en outre un affichage de données de zone quantitatives relatives aux cellules tumorales ou des zones de cellules tumorales qui sont classées par rapport à des scores de Gleason.

15. Système selon l'une quelconque des revendications 11 à 14, comprenant en outre une station de travail (140) associée au microscope possédant un dispositif d'affichage ;

dans lequel le dispositif d'affichage fournit des outils pour un utilisateur de la station de travail pour dessiner une annotation sur une image de la vue, et dans lequel l'annotation est sauvegardée avec l'image de la vue dans une mémoire d'ordinateur ; et/ou

dans lequel le dispositif d'affichage fournit un accès aux outils pour personnaliser la présentation de l'amélioration sur le champ de vue.

# Fig. 1

## Fig. 2A

150

152

## Fig. 2B

152

150

154

## Fig. 3A

152

150

## Fig. 3B

87% Gleason 3
13% Gleason 4
0.12μm diameter

156

158

## Fig. 4A

150

## Fig. 4B

150

156

# Fig. 5

ML pattern recognizer
(Deep CNN)

202 — Memory

200

204 — CPU

206 — Graphics Card

208

210

212 — Memory

126

214 — Inference accelerator

220

216

To AR display          To workstation

# Fig. 6

New slide inserted
into microscope — 302

300

Specimen classifier
or manual selection picks
mode and ML model
(e.g. prostate cancer, breast
cancer, malaria detection) — 304

306

Send image of FoV to compute unit

Update

Slide moved
(panning) — 308

Update

Objective changed
(zooming) — 310

Run inference — 312

Project augmentation into FoV — 314

# Fig. 7

22

| | |
|---|---|
| Dark Red → | 1.0 |
| | 0.9 |
| | 0.8 |
| Orange → | |
| | 0.7 |
| Yellow → | |
| | 0.6 |
| | 0.5 |
| | 0.4 |
| Green → | |
| | 0.3 |
| Blue → | 0.2 |
| Dark Blue → | 0.1 |
| Violet → | 0.0 |

≥0.9 Patch has the highest likelihood to have cancer cells

≥0.5 but <0.9: Patch looks more abnormal than normal, but does not have high enough confidence the presence or absence of metastasis

This may occur with patches that contain isolated tumor cells

≥0.1 but <0.5: Patch looks more normal than abnormal, but does not have high enough confidence the presence or absence of metastasis

This may occur with patches that contain isolated tumor cells

<0.1: Patch is likely to be normal

# Fig. 8

200

206 — Graphics card

250 — Combiner function

Output 408A   Output 408B   Output 408C   Output 408D

406A   406B   406C   406D

Input 404A   Input 404B   Input 404C   Input 404D

Independent deep CNN pattern recognizers pre-trained on slide set

40X   20X   10X   5X

402A   402B   402C   402D

299x299 patch at different scales (magnifications)

# Fig. 9

micro SD
512 GB
BREAST TISSUE — 902

micro SD
512 GB
PROSTATE TISSUE — 904

. . . .

900

micro SD
512 GB
TUBERCULOSIS
Mycobacterium — 906

. . . .

micro SD
512 GB
Plasmodium — 908

. . . .

# Fig. 10

128 (Fig. 1)

P

124

104

BS2

PI

Teaching module 2

SI

101

BS1

Teaching module 1

S

106

120

# Fig. 11A
### deep learning algorithm development

whole slide scanned image     label

patch of interest
32x32 μm
-0.21 μm/pixel @ 40X

□ label for patch of interest [0,1]

context
100x100 μm
-0.21 μm/pixel @ 40X

rescale to match microscope resolution

context
100x100 μm
(911 x 911 pixels)
-0.21 μm/pixel @ 40X

[0,1]

algorithm training

1100

# Fig. 11B
### deep learning algorithm application

frame grabbing

image projection as overlay

1102

1101

prediction heatmap

1100    algorithm inference

## Fig. 11C

**Frame Number**

Software Pipeline

CPU

Frame Grabber

Host

| | Grab Frame #3 | Demosaic Frame #3 | Prepare Frame #3 | Run Model #3 | Generate Heartmap #3 | Display Results #3 | | |
|---|---|---|---|---|---|---|---|---|

Time

T1  T2  T3  T4  T5  T6  T7  T8

# Fig. 12A

4X 10X 20X 40X

Lymph mode

# Fig. 12B

4X 10X 20X

Prostate

# Fig. 13

## Fig. 14A

## Fig. 14B

## Fig. 14C

## Fig. 14D

## Fig. 14E

## Fig. 14F

# Fig. 15

drawing
interface for
lightweight
annotations

Prostate
10x

line thickness

brightness

EP 3 776 458 B1

# Fig. 16

# Fig. 17

# Fig. 18

# Fig. 19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62656557
- US 2017169276 A1
- US 20160183779
- WO 2016130424 A1
- US 2019012674 W

- US 17019051 W
- US 83945215, C. Szegedy
- US 39553016, C. Vanhoucke
- US 20160342891, Jonathon Ross

**Non-patent literature cited in the description**

- **G. LITJENS et al.** *Deep learning as a tool for increasing accuracy and efficiency of histopathological diagnosis,* May 2016, vol. 6, 26286
- **D. WANG et al.** Deep Learning for Identifying Metastatic Breast Cancer. *arXiv:1606.05718v1,* June 2016
- **A. MADABHUSHI et al.** Image analysis and machine learning in digital pathology: Challenges and opportunities. *Medical Image Analysis,* 2016, vol. 33, 170-175
- **A. SCHUAMBERG et al.** H&E-stained Whole Slide Deep Learning Predicts SPOP Mutation State in Prostate Cancer. *bioRxiv preprint, http:/.bioRxiv.or/content/early/2016/07/17/064279*
- **QUINN et al.** Deep Convolutional Neural Networks for Microscopy-based Point of Care Diagnostics. *Proceedings of International Conference on Machine Learning for Health Care,* 2016
- **WATSON et al.** Augmented microscopy: real-time overlay of bright-field and near-infrared fluorescence images. *Journal of Biomedical Optics,* October 2015, vol. 20 (10

- **WATSON et al.** Augmented microscopy: real-time overlay of bright-field and near-infrared fluorescence images. *Journal of Biomedical optics,* October 2015, vol. 20 (10
- **EDWARDS et al.** Augmentation of Reality Using an Operating Microscope. *J. Image Guided Surgery.,* 1995, vol. 1 (3
- Stereo augmented reality in the surgical microscope. **EDWARDS et al.** Medicine Meets Virtual Reality. IOS Press, 102
- **C. SZEGEDY et al.** Going Deeper with Convolutions. *arXiv: 1409.4842,* September 2014
- **C. SZEGEDY et al.** Rethinking the Inception Architecture for Computer Vision. *arXiv:1512.00567,* December 2015
- **C. SZEGEDY et al.** Inception-v4, Inception-ResNet and the Impact of Residual Connections on Learning. *arXiv:1602.0761,* February 2016
- **G. LITJENS et al.** *Deep learning as a tool for increasing accuracy and efficiency of histopathological diagnosis,* May 2016